(19) Europäisches Patentamt
European Patent Office
Office européen des brevets

(11) EP 2 815 738 A1

(12) **EUROPEAN PATENT APPLICATION**
published in accordance with Art. 153(4) EPC

(43) Date of publication:
**24.12.2014 Bulletin 2014/52**

(21) Application number: **13748798.9**

(22) Date of filing: **13.02.2013**

(51) Int Cl.:
***A61K 8/36*** (2006.01) ***A61K 8/14*** (2006.01)
***A61K 8/34*** (2006.01) ***A61K 8/362*** (2006.01)
***A61K 8/365*** (2006.01) ***A61K 8/37*** (2006.01)
***A61K 8/41*** (2006.01) ***A61K 8/44*** (2006.01)
***A61Q 5/12*** (2006.01) ***B01J 13/04*** (2006.01)

(86) International application number:
**PCT/JP2013/053397**

(87) International publication number:
**WO 2013/122102 (22.08.2013 Gazette 2013/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.02.2012 JP 2012028294**
**18.07.2012 JP 2012159626**

(71) Applicant: **Kao Corporation**
**Chuo-ku**
**Tokyo 103-8210 (JP)**

(72) Inventors:
- **FUJII Kenko**
**Tokyo 131-8501 (JP)**
- **MIYATANI Tsukasa**
**Tokyo 131-8501 (JP)**
- **AOYAGI Kensuke**
**Tokyo 131-8501 (JP)**

(74) Representative: **Hoffmann Eitle**
**Patent- und Rechtsanwälte PartmbB**
**Arabellastraße 30**
**81925 München (DE)**

(54) **METHOD FOR PRODUCING VESICLE COMPOSITION**

(57) Provided is a method for producing a vesicle composition having an aqueous phase as a continuous phase, containing: an oil phase-preparation step for dissolving an oil phase containing components (A) to (D) at a temperature of equal to or higher than melting point of the oil phase, the components (A) to (D) being: the component (A): a fatty acid of total carbon number of from 12 to 40; the component (B): a tertiary amine compound of total carbon number of from 8 to 75; the component (C): an organic acid of total carbon number of from 1 to 10; and the component (D): the first polyol, an emulsification step for adding the aqueous phase to the oil phase obtained in the oil phase-preparation step while being mixed; an addition step for adding a component (D'): the second polyol, after a quantity of the aqueous phase added to the oil phase is equal to or larger than 1 time by mass of a quantity of the oil phase; and a cooling step for cooling the obtained composition after the emulsification step.

EP 2 815 738 A1

## Description

TECHNICAL FIELD

The present invention relates to a method for producing a vesicle composition, a vesicle composition, a hair cosmetic composition and a method for producing a hair cosmetic composition.

BACKGROUND ART

In recent years, utilizations of permanents, hair colors, hair bleaches and the like are generalized, and on the other hand, damages of hair associated with such chemical treatments are problematic. Hair cosmetic compositions such as rinses, conditioners, treatments and the like are conventionally used for improving feels of hairs after shampooing, and further improvements in performances of the compositions are expected in view of reducing the damages of the hairs.
Patent Document 1 proposes a hair cosmetic composition containing specific types of branched fatty acids, which is related to a technology for restraining damages of hairs and providing good feels.
Patent Document 2 proposes a hair cosmetic composition containing a vesicle composition, which is prepared by combining specific organic acids in addition to a specific tertiary amine and a specific branched fatty acid. Patent Document 2 describes that a spread feel in applying and a smoothness in the rinsing, which have been achieved by the conventional hair cosmetic composition, can be maintained or improved, even if the content of branched fatty acid is reduced as compared with that of the conventional hair cosmetic composition.
Meanwhile, an emulsion phase inversion method and a liquid crystal emulsion method are known as methods for preparing emulsified compositions. The emulsion phase inversion method is a process to carry out an emulsification while an aqueous phase is added to an oil phase. The liquid crystal emulsion method is a process to carry out an emulsification while adding an aqueous phase to a liquid crystalline phase.
Patent Document 3 discloses a liquid crystal emulsion phase inversion method for producing an emulsified liquid by creating a liquid crystal structure while adding an aqueous phase in an oil phase and then further adding the aqueous phase in the liquid crystal structure to cause phase inversion. Patent Document 3 describes that improved emulsified liquid is obtainable by suitably controlling a stirring energy, which can be calculated with a power consumption and a stirring time in the formation of the liquid crystal structure.

RELATED DOCUMENTS

PATENT DOCUMENTS

[Patent Document 1]
Japanese Laid-Open Patent Publication No. H04-173719 (1996)
[Patent Document 2]
WO 2011/007525
[Patent Document 3]
Japanese Laid-Open Patent Publication No. 2007-161683

SUMMARY OF THE INVENTION

The present invention is directed to provide the following producing method. Provided is a method for producing a vesicle composition having an aqueous phase as a continuous phase, the method containing: an oil phase-preparation step for dissolving components (A) to (D) at a temperature of equal to or higher than melting point of the oil phase, the components (A) to (D) being: the component (A): a fatty acid of total carbon number of from 12 to 40; the component (B): a tertiary amine compound of total carbon number of from 8 to 75; the component (C): an organic acid of total carbon number of from 1 to 10; and the component (D): the first polyol;
an emulsification step for adding the aqueous phase to the oil phase obtained in the oil phase-preparation step while being mixed;
an addition step for adding a component (D'): the second polyol, after a quantity of the aqueous phase added to the oil phase is equal to or larger than 1 time by mass of a quantity of the oil phase; and
a cooling step for cooling the obtained composition after the emulsification step.

DETAILED DESCRIPTION OF THE INVENTION

[First Embodiment]

While relatively larger particle size of the vesicle is obtainable in the case that the vesicle is produced according to the method described in the above-noted Patent Document 2, there are still rooms for improvements in the storage stability of the vesicle, and more specifically in the storage stability under the conditions of lower temperature.

The present embodiment is directed to vesicle compositions having enhanced storage stability at lower temperature and hair cosmetic compositions containing thereof.

Present inventors have eagerly conducted their investigations in order to improve the storage stability of the vesicle composition, and found that the storage stability of the obtainable vesicle composition is greatly improved by producing the vesicle composition under specific production conditions. More specifically, it was found that, when a vesicle composition containing a fatty acid, a tertiary amine and an organic acid is produced, a polyol is blended in an oil phase and subsequently the same or a different type of polyol is further added therein during the production method to achieve further improvement in the storage stability at lower temperature.

According to the present embodiment, a vesicle composition having enhanced storage stability at the lower temperature condition can be produced.

Here, respective process steps and respective components used therein will be specifically described below. In the beginning, the components (A) to (D') will be described.

The component (A) is a fatty acid of total carbon number of from 12 to 40. More specifically, total carbon number of the component (A) is equal to or larger than 12 and equal to or smaller than 40. While the component (A) may be of a saturated fatty acid or an unsaturated fatty acid, saturated fatty acid is more preferable in view of an oxidation stability in a product. More specifically, the component is of one, two or more fatty acid(s) represented by any one of the following general formula (1) and general formula (2).

[General formula 1] $H_3C\text{-}(CH_2)_n\text{-}COOH$ (1)

(In the above-described General formula (1), n represents an integer number of from 10 to 38).

[General formula 2]

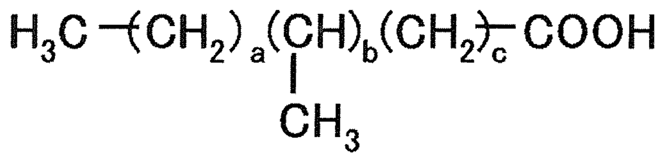

(2)

(In the above-described General formula (2), the sum of a, b and c is: a+b+c = 9 to 37; and b is 1).

The linear fatty acid of the component (A) represented by General formula (1) may further be of total carbon number of equal to or larger than 14, in view of creating the vesicle with further sureness, and more preferably of equal to or larger than 16, and on the other hand, may be of total carbon number of equal to or smaller than 30, and preferably of equal to or smaller than 24, and further preferably equal to or smaller than 22. More specifically, one, two or more selected from myristic acid, palmitic acid, stearic acid, arachidic acid and behenic acid may be employed, and among these, one, two or more selected from palmitic acid, stearic acid, arachidic acid and behenic acid may be preferably employed.

Branched fatty acid represented by General formula (2) may be of total carbon number of equal to or larger than 12 and preferably of equal to or larger than 16, and on the other hand, may be of total carbon number of equal to or smaller than 40 and preferably of equal to or smaller than 22, and further preferably equal to or smaller than 20. To summarize these aspects, total carbon number of the branched fatty acid represented by General formula (2) may be preferably of from 12 to 40, and further preferably 16 to 22, and more preferably 16 to 20. More specifically, one, two or more selected from 2-heptyl undecanoic acid (isostearic acid), 14-methyl pentadecanoic acid, 15-methyl hexadecanoic acid, 16-methyl heptadecanoic acid, 17-methyl octadecanoic acid, 18-methyl nonadecane acid, 19-methyl eicosanic acid, 20-methyl heneicosanoic acid, 14-methyl hexadecanoic acid, 15-methyl heptadecanoic acid, 16-methyl octadecanoic acid, 17-methyl nonadecane acid, 18-methyl eicosanic acid and 19-methyl heneicosanoic acid may be employed.

In addition, branched fatty acids, which are described in "Cosmetics Raw Material Standards", the Second Edition, Explanatory Note I (1984)," YAKUJI NIPPO LIMITED, pp. 87 (C) "Isostearic acid", may be employed. It is described that such branched fatty acids are C18 branched-chain fatty acids, which have methyl group composing the

side chain though the position is not specified and are obtainable by conducting hydrogenation to unsaturated side chain fatty acid, which has been created as a byproduct in the synthesis of dimer acids from oleic acid. More specifically, ISOSTEARIC ACID EX (commercially available from KOKYU ALCOHOL KOGYO CO., LTD.) may represent this compound.

The branched fatty acid represented by General formula (2) may be separated and extracted from hairs in accordance with, for example, the description of LIPIDS, vol. 23, No. 9, 878 to 881 (1988), or alternatively, may be synthesized according to the descriptions of Patent Document 1 described above in the section of BACKGROUND ART.

Typical extracted product may include an extracted material from lanolin, more specifically, lanolin fatty acid and its salt. The lanolin fatty acid contains methyl-branched long chain fatty acid, which is referred to as iso fatty acid or anteiso fatty acid, at the amount of about 50 % wt. More specifically CRODACID 18-MEA (commercially available from Croda Japan KK), SKLIRO (commercially available from Croda Japan KK), FA-NH (commercially available from Nippon Fine Chemical Co., Ltd.) are exemplified.

Respective two or more types of the fatty acids represented by General formula (1) or General formula (2) may be employed for the component (A), or two or more types of the fatty acids represented by different General formulae may alternatively be jointly employed, and it is preferable in view of obtaining smoothness and suppressing pastiness after the drying to jointly employ those represented by General formula (1) and those represented by General formula (2). When those represented by General formula (1) and those represented by General formula (2) are jointly employed, larger particle diameter of the vesicle of, for example, equal to or larger than 5 μm is obtainable, and thus, in view of obtaining the smoothness in the rinsing, depressing the slimy feel, obtaining the smoothness after the drying and depressing the pastiness, those represented by General formula (1) and those represented by General formula (2) may be preferably jointly employed at the mass ratio therebetween: (1)/(2) = 70/30-40/60. More specifically, it is preferable that (1)/(2) is equal to or larger than 40/60 and is equal to or lower than 70/30.

The content of the component (A) is preferably equal to or higher than 0.1 % by mass in the vesicle composition, and is more preferably equal to or higher than 1 % by mass. On the other hand, it is preferably equal to or lower than 10 % by mass, and is more preferably equal to or lower than 5 % by mass.

In the next, the component (B) will be described.

The component (B) is a tertiary amine compound of total carbon number of from 8 to 75, and more specifically is one, two or more of tertiary amine compound(s) represented by General formulae (3) to (6). Total carbon number of the component (B) is equal to or larger than 8 and equal to or smaller than 75.

[General formula 3]

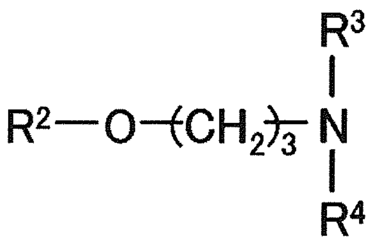

(3)

[General formula 4]

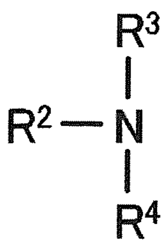

(4)

[General formula 5]

$$R^5-CONH-(CH_2)_l-N-(R^6)_2 \qquad (5)$$

[General formula 6]

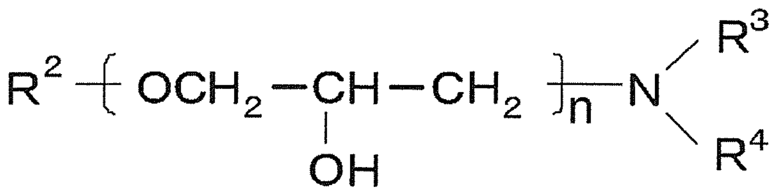

(6)

In the above-described General formulae (3), (4) and (6), $R^2$ represents linear or branched alkyl group or alkenyl group of carbon number of from 6 to 24, $R^3$ and $R^4$ are same or different alkyl group(s) of carbon number of from 1 to 6 or $(AO)_mH$ (AO is oxy alkylene group of carbon number of from 2 to 4, m represents an integer number of from 1 to 6, m of AO may be the same or different and the sequence thereof is arbitrary). Also, n in General formula (6) represents an integer number of from 1 to 5. Also, in the above-described general formula (5), $R^5$ represents aliphatic hydrocarbon group of carbon number of from 11 to 23, $R^6$, each of which may be same or different, represents hydrogen atom or alkyl group of carbon number of from 1 to 4, and 1 represents an integer number of from 2 to 4.

In relation to tertiary amine compounds of the component (B) represented by General formula (3), General formula (4) and General formula (6), $R^2$ preferably represents linear or branched alkyl group or alkenyl group having carbon number of equal to or larger than 12, more preferably represents linear or branched alkyl group or alkenyl group of carbon number of equal to or larger than 14, and further preferably represents linear alkyl group of carbon number of equal to or larger than 18 in these formulae.

Also, in General formula (3), General formula (4) and General formula (6), $R^2$ is preferably linear or branched alkyl group or alkenyl group having carbon number of equal to or lower than 24 and is further preferably linear alkyl group having carbon number of equal to or lower than 22, in view of providing the smoothness in the rinsing and providing the smoothness after the drying.

Further, in General formula (3), General formula (4) and General formula (6), one of $R^3$ and $R^4$ may be preferably alkyl group having carbon number of from 1 to 6, and may be more preferably methyl group or ethyl group, and further, it is more preferable that the both are the same.

More specifically, as long as such compound is a tertiary amine compound of the component (B) represented by General formula (3), one or two selected from N,N-dimethyl-3-hexadecyl oxypropyl amine and N,N-dimethyl-3-octadecyl oxypropyl amine (also referred to as "N,N-dimethyl-octadecyloxypropyl amine") may be employed.

Also, as long as such compound is a tertiary amine compound of the component (B) represented by General formula (4), one or two selected from N,N-dimethyltetradecylamine, N,N-dimethylhexadecylamine, N,N-dimethylbehenylamine and N,N-dimethyl-n-octadecylamine may be more specifically employed.

As long as such compound is a tertiary amine compound of the component (B) represented by General formula (6), one or two selected from hexadecyloxy (2-hydroxypropyl)dimethylamine and octadecyloxy (2-hydroxypropyl)dimethylamine may be more specifically employed.

In the tertiary amine compound of the component (B) represented by General formula (5), $R^5$ preferably represents linear or branched alkyl group or alkenyl group having carbon number of equal to or larger than 13, and further preferably represents linear or branched alkyl group or alkenyl group having carbon number of equal to or larger than 17.

On the other hand, $R^5$ preferably represents linear or branched alkyl group or alkenyl group having carbon number of equal to or lower than 23.

As tertiary amine compound represented by General formula (5), one or two selected from N-(3-(dimethylamino)propyl) docosaneamide and N-(3-(dimethylamino)propyl) stearamide may be more specifically employed.

A single tertiary amine of the component (B) may be employed alone, or two or more thereof may be jointly employed. The component (B) may be preferably one, two or more selected from amidoamines, etheramines, alkylamines and hydroxyetheramines, and may be further preferably one, two or more selected from amidoamines and etheramines, and may be more preferably one, two or more selected from etheramines.

In addition, in view of achieving enhanced storage stability at higher temperature, etheramines of General formula (3) and amidoamines of General formula (5) are preferable as the tertiary amine of the component (B).

The content of the component (B) may be preferably 0.5 % by mass in the vesicle composition in view of

providing the smoothness in the rinsing and providing the smoothness after the drying, and further preferably equal to or higher than 1 % by mass, and more preferably equal to or higher than 1.5 % by mass. On the other hand, the content of the component (B) may be preferably equal to or lower than 15 % by mass in the vesicle composition in view of providing the smoothness in the rinsing and providing the smoothness after the drying, and further preferably equal to or lower than 10 % by mass, and more preferably equal to or lower than 7 % by mass.

Further, the content of the component (B) may be preferably from 0.5 to 15 % by mass in the vesicle composition, in view of providing the smoothness in the rinsing and providing the smoothness after the drying. Further, from 1 to 10 % by mass is preferable, and from 1.5 to 7 % by mass is more preferable.

In the next, the component (C) will be described.

The component (C) is an organic acid having total carbon number of from 1 to 10. More specifically, total carbon number of the component (C) is equal to or larger than 1 and equal to or smaller than 10. In view of providing the smoothness in the rinsing and providing the smoothness after the drying, total carbon number of the organic acid may be preferably equal to or larger than 1, and more preferably equal to or larger than 2. On the other hand, in view of providing the smoothness in the rinsing and providing the smoothness after the drying, total carbon number of the organic acid may be preferably equal to or smaller than 8, and more preferably equal to or smaller than 6.

Such organic acid may be more specifically one, two or more selected from: monocarboxylic acids such as acetic acid, propionic acid, caprylic acid, capric acid and the like; dicarboxylic acids such as malonic acid, succinic acid, glutaric acid, adipic acid, maleic acid, fumaric acid and the like; hydroxy carboxylic acids such as glycolic acid, lactic acid, hydroxy acrylic acid, glyceric acid, malic acid, tartaric acid, citric acid and the like; aromatic carboxylic acids such as benzoic acid, salicylic acid, phthalic acid and the like; and acidic amino acids such as glutamic acid, asparagic acid and the like. Among these, it is preferable to employ one, two or more selected from hydroxy carboxylic acids and acidic amino acids. Glycolic acid, citric acid, lactic acid and malic acid are further preferable for the hydroxy carboxylic acids, and glycolic acid, lactic acid and malic acid are more preferable, and glycolic acid and lactic acid are even more preferable. Glutamic acid is further preferable as the acidic amino acids.

The content of the component (C) may be preferably equal to or higher than 0.05 % by mass in the vesicle composition, in view of providing the smoothness in the rinsing and providing the smoothness after the drying, and may be more preferably equal to or higher than 0.1 % by mass. On the other hand, the content of the component (C) may be preferably equal to or lower than 4 % by mass in the vesicle composition in view of the handling of the vesicle composition and the storage stability, and may be more preferably equal to or lower than 2 % by mass.

Next, the component (D) and the component (D') will be described.

The component (D) and the component (D') are the first and the second polyols, respectively. The polyols employed as the component (D) or the component (D') may be more specifically one, two or more compound(s) selected from ethylene glycol, diethylene glycol, propylene glycol, dipropylene glycol, 1,3-propanediol, glycerol, 1,3-butylene glycol and isopentyl diol, and polypropylene glycol which is in the state of liquid at 25 degrees C and sorbitol. Further, dihydric alcohols are preferable, and propylene glycol and dipropylene glycol are more preferable. Also, the component (D') may be preferably one, two or more compound(s) selected from ethylene glycol, diethylene glycol, propylene glycol, dipropylene glycol, glycerol and the like, and may be further preferably one, two or more compound(s) selected from propylene glycol and dipropylene glycol.

The component (D) may be the same as, or different from, the component (D').

The content of the component (D) may be preferably equal to or higher than 0.5 % by mass over the whole vesicle composition in view of the storage stability of the vesicle composition, and more preferably equal to or higher than 1 % by mass, and further preferably equal to or higher than 2 % by mass. On the other hand, the content of the component (D) may be preferably equal to or lower than 40 % by mass, and more preferably equal to or lower than 30 % by mass, and further preferably equal to or lower than 20 % by mass.

In addition, it is preferable to prepare an oil phase in the oil phase-preparation step in the method for producing the vesicle composition as will be discussed later, so that the concentration of the component (D) in the obtainable vesicle composition falls within the above-described range.

Two or more types of polyols may be jointly employed for the component (D') in view of providing improved storage stability at lower temperature and improved feel of the smoothness in the rinsing. For example, when two types of polyols are employed, it is preferable to employ dipropylene glycol and propylene glycol together.

Further, when two types of polyols are jointly employed, dipropylene glycol and propylene glycol may be preferably employed by the weight ratio of from 10:90 to 90:10, and more preferably at from 20:80 to 80:20, in view of providing further improved storage stability at lower temperature.

In addition, the content of the component (D') may be preferably equal to or higher than 1 % by mass over the whole vesicle composition, and more preferably equal to or higher than 3 % by mass, and further preferably equal to or higher than 5 % by mass. On the other hand, the content of the component (D') may be preferably equal to or lower than 30 % by mass over the whole vesicle composition, and more preferably equal to or lower than 20 % by mass, and further preferably equal to or lower than 15 % by mass.

The content of the component (D') within the above-described range is preferable in view of the storage stability and the sense of feel.

Also, in the step of the addition of the component (D') in the method for producing the vesicle composition as will be discuss later, it is preferable to carry out the addition so that the concentration of the component (D') in the obtainable vesicle composition falls within the above-described range.

The component (A), the component (B), the component (C), the component (D) and water, and optionally the component (D') as required, create the vesicle, and more specifically, a type of a vesicle composition, in which multiple-layered lamella vesicles (so-called onion vesicles) composed of several dual-membranes are dispersed in water, is easily formed. While the vesicle ordinarily refers to coacervates, the internal layer of which is hollow or of aqueous phase, the multiple-layered lamella vesicles created here include those having structures, the internal layer of which is partially or wholly of oil phase. The "vesicle" also includes the multiple-layered lamella vesicle in the present application.

In view of providing the smoothness in the rinsing and providing the smoothness after the drying, the molar ratio (A)/(C) of the component (A) and the component (C) is preferably equal to or higher than 5/5, and further, is preferably equal to or higher than 7/3, and on the other hand, is preferably equal to or lower than 9/1, and further is preferably equal to or lower than 8/2. Also, in view to achieving increased vesicle volumetric concentration in the vesicle dispersion solution, it is preferable that the molar ratio (A)/(C) of the component (A) and the component (C) falls with the above-described range.

Also, in view of achieving effective contribution of the component (A), the component (B) and the component (C) to the vesicle formation, ratio [((A)+(C))/(B)] of the sum of an acid equivalents of the component (A) and the component (C) (also referred to as "acid equivalents of (A)+(C)") over a base equivalent of the component (B) is preferably equal to or higher than 0.25, and is more preferably equal to or higher than 0.5, and is further preferably equal to or higher than 0.6. On the other hand, [((A)+(C))/(B)] is preferably equal to or lower than 4, and is more preferably equal to or lower than 2, and is further preferably equal to or lower than 1.8.

Further, in view of the storage stability and the handling ability of the vesicle dispersion solution, the sum of the component (A), the component (B) and the component (C) in the vesicle dispersion solution or in other words in the vesicle composition is preferably equal to or higher than 1 % by mass, and on the other hand is more preferably equal to or lower than 20 % by mass and further preferably equal to or lower than 15 % by mass.

In the next, the respective steps in the method for producing the vesicle composition will be further specifically described. The vesicle dispersion solution may be preferably prepared by conducting, for example, the following steps, which are more specifically composed of:

an oil phase-preparation step for dissolving the oil phase containing the component (A), the component (B), the component (C) and the component (D) at a temperature of equal to or higher than melting point of the oil phase;
an emulsification step for adding an aqueous phase in the oil phase obtained in the oil phase-preparation step while being mixed;
an addition step for adding a component (D'): the second polyol, after a quantity of the aqueous phase added to the oil phase is equal to or larger than 1 time by mass of the oil phase; and
a cooling step for cooling the obtained composition to a temperature that is equal to or lower than the gel transition temperature of the vesicle after the emulsification step. According to such procedure, the vesicle composition containing the continuous phase composed of the aqueous phase is obtainable. It is also preferable that the obtainable vesicle composition is in the form of the dispersion solution of the vesicle (premix).

First of all, it is necessary that the oil phase is dissolved in the oil phase-preparation step in view of the stable production. For this reason, it is preferable to achieve the dissolution at a temperature that is equal to or higher than the melting point of the oil phase, and is further preferable to achieve the dissolution at a temperature that is equal to or higher by 5 degrees C than the melting point of the oil phase, and is more preferable to achieve the dissolution at a temperature that is equal to or higher by 10 degrees C than the melting point of the oil phase.

Also, it is preferable that the oil phase is in the condition of being uniformly mixed. Thus, it is preferable to carry out the dissolution in the present step while mixing the oil phase. While the mixing process is not limited to any specific method, it is preferable to mix by, for example, stirring.

In the next, in the emulsification step, an aqueous phase is dripped to the oil phase dissolved in the oil phase-preparation step, which is more specifically the oil phase in the shear-mixed condition. While the available mixing equipment is not limited as long as the shear-mixing can be achieved, if the mixture exhibits higher viscosity in the process of adding the aqueous phase, it is preferable to employ an apparatus that is applicable to the mixing of highly viscous products, such as, for example: AGI HOMO MIXER and TK COMBI MIX, commercially available from PRIMIX Corporation; Vacuum Emulsification Stirring Apparatus, commercially available from MIZUHO Industrial Co., Ltd.; Maxblend, commercially available from Sumitomo Heavy Industries, Ltd.; Super Mix Stirring Vessel commercially available from Satake Chemical Equipment Mfg Ltd., and the like.

Optimum values of the dripping rate of the aqueous phase to the oil phase and the stirring rate in the dripping depend upon the formulation and the component ratio of the vesicle composition and the size and the shape of the blending vessel. The stirring rate may be suitably selected as long as the vesicle can be formed under the condition of the selected rate. Although it is not intended to limit the stirring rate, it is preferable to stir at a rate of from 20 rpm to 1000 rpm in, for example, a blending vessel having a capacity of 2 L. Also, the stirring rate in the emulsification in the emulsification step may be, for example, equal to or higher than 15 rpm, and may be preferably equal to or higher than 20 rpm, and on the other hand for example may be equal to or lower than 800 rpm, and may preferably be equal to or lower than 500 rpm, and may be further preferably equal to or lower than 200 rpm.

The dripping rate of the aqueous phase in the oil phase may be suitably selected, and it is preferable to take longer time such as ten minutes or longer to carry out the dripping, in view of effectively creating the desired structure. Although it is not intended to limit the dripping rate, it is preferable to carry out the dripping at a rate of 12 to 50 g/minute if the whole quantity of the dripping aqueous phase is, for example, 1,400 g. Also, the time for dripping the aqueous phase during the emulsification in the emulsification step may be, for example, equal to or longer than 5 minutes and equal to or shorter than 100 minutes.

Also, the mixing may be carried out in the emulsification step such that the maximum value of a power consumption Pv is equal to or higher than 0.1 $kW/m^3$ and equal to or lower than 10 $kW/m^3$. Meanwhile, Pv is an indicator for evaluating the degree of mixing of the liquid in the blending vessel, which is determined generally by geometric factors of the a blending vessel and the impeller, rotational speed of the impeller, density, viscosity and volume of the liquid in the blending vessel and the like. Further detailed descriptions for Pv will be made in Second Embodiment. In addition to above, according to the viewpoints described in Second embodiment as will be discussed later, it is preferable to carry out the aforementioned mixing, so that the maximum value of the power consumption Pv is equal to or higher than 0.1 $kW/m^3$, is preferably equal to or higher than 0.5 $kW/m^3$ and is more preferably equal to or higher than 0.8 $kW/m^3$, and on the other hand, is equal to or lower than 10 $kW/m^3$, is preferably equal to or lower than 7 $kW/m^3$, is more preferably equal to or lower than 5 $kW/m^3$, and preferably equal to or lower than 3 $kW/m^3$.

Also, a phase inversion emulsification may be conducted in the emulsification step by carrying out the mixing under the condition that the total weight of the oil phase and the aqueous phase for loading in the blending vessel is equal to or larger than, for example, 10 kg. In addition to above, according to the viewpoints described in Second Embodiment as will be discussed later, the total weight of the oil phase and the aqueous phase for loading in the blending vessel may be more preferably equal to or larger than 100 kg, and further preferably equal to or larger than 200 kg.

While the temperature in dripping of the aqueous phase in the emulsification step can be suitably determined on the basis of the temperature of the oil phase and the temperature of the aqueous phase to be dripped and the levels of the heating and the cooling in the mixing equipment, it is preferable to establish the temperature of the oil phase and the temperature of the aqueous phase to be dripped so as to be equal to or higher than a gel transition temperature of the vesicle to be formed. More specifically, the temperature during the mixing in the emulsification step may be preferably equal to or higher than the gel transition temperature of the vesicle to be obtained. As described above, the procedure for the emulsification, in which the aqueous phase is dripped while stirring the oil phase, is generally referred to as a phase inversion emulsification. In the present embodiment, the oil phase is constituted as described above to create the vesicle composition by the phase inversion emulsification, such that the vesicle composition acquires enhanced handling ability.

Here, pure water such as deionized water, distilled water or the like may be preferably employed for the "aqueous phase." While the quantity of the aqueous phase may be arbitrarily selected as long as the vesicle can be created, such quantity may be equal to or higher than 1 time by mass of the oil phase, may be preferably equal to or larger than twice by mass of the oil phase, and may further preferably be equal to or larger than 3 times by mass thereof. On the other hand, it may be equal to or lower than 100 times by mass of the oil phase, and more preferably equal to or lower than 50 times by mass of the oil phase.

The addition step for adding the component (D') may be conducted in any time after the quantity of the aqueous phase added in the oil phase that has been prepared in the oil phase-preparation step reaches equal to or larger than 1 time by mass of the oil phase. For example, in the emulsification step, the aqueous phase containing the component (D') blended therein may be added after the quantity of the aqueous phase that is equal to or larger than 1 time by mass of the oil phase has been added while mixing, or alternatively, only the component (D') may be added after all the aqueous phase has been added. When the component (D') is added after all the aqueous phase has been added, the timing of the addition of the component (D') may be after or before the cooling step. The addition step for adding the component (D') may be conducted after, for example, the emulsification step, and may be preferably conducted after the cooling step.

In view of providing the storage stability of the obtainable vesicle composition and the smoothness in the rinsing and the smoothness after the drying, the addition step may be preferably conducted after that the quantity of the aqueous phase is as twice or more by mass as the quantity of the oil phase, and more preferably conducted after that the quantity of the aqueous phase is equal to or larger than 3 times by mass of the quantity of the oil phase. Further, it is more

preferable to conduct after the emulsification step, and further preferably after the cooling step as will be discussed later.

In addition, concerning the temperature in adding the component (D'), it is preferable that the temperature in the blending vessel at the start of the addition is equal to or higher than 10 degrees C, in view of providing improved productivity, and more preferably equal to or higher than 20 degrees C. In addition, in view of the storage stability of the vesicle composition, the temperature in the blending vessel at the start of the addition is preferably equal to or lower than 85 degrees C, and is more preferably equal to or lower than 50 degrees C.

Further, the temperature of the component (D') at the start of the addition is preferably equal to or higher than 10 degrees C, and is more preferably equal to or higher than 20 degrees C.

In addition, in view of the storage stability of the vesicle composition, the temperature of the component (D') at the start of the addition is preferably equal to or lower than 85 degrees C, and is more preferably equal to or lower than 50 degrees C.

In the cooling step, the vesicle composition, which has been obtained in the emulsification step, is cooled, from the viewpoint of the stability of the obtainable vesicle composition. The cooling temperature is preferably equal to or lower than the gel transition temperature of the vesicle composition.

According to the above-described procedure, the vesicle composition is obtainable. In the method of the present embodiment, the first polyol of the component (D) is preliminarily blended in the oil phase in the oil phase-preparation step, and then the blended mixture is dissolved, and after that, the addition of the aqueous phase in the emulsification step is conducted. Then, after the stage where the addition of the aqueous phase is advanced to some extent, the addition step for adding the second polyol of the component (D') is conducted. Further, after the addition of the aqueous phase in the emulsification step, the cooling step is conducted. These respective steps may be combined according to a specific sequence to provide improved storage stability of the obtainable vesicle. Concerning the storage stability, for example, a vesicle composition having enhanced storage stability at lower temperature of around -10 degrees C can be obtained. In addition, for example, a vesicle composition having well-balanced storage stability including enhanced storage stability at higher temperature of around 50 degrees C and enhanced storage stability at lower temperature of around -10 degrees C can also be obtained.

In addition, the mean particle diameter of the vesicle contained in the vesicle composition is preferably within the range described in Second Embodiment as will be discussed later. More specifically, in view of providing further improved spread feel in applying over the hair, the mean particle diameter may be, for example, equal to or larger than 2 μm, preferably equal to or larger than 3 μm, and further preferably equal to or larger than 5 μm, and on the other hand, is for example, equal to or smaller than 20 μm, preferably equal to or smaller than 18 μm, and further preferably equal to or smaller than 15 μm. Here, the mean particle diameter may be measured as the volumetric mean particle diameter by employing, for example, a particle size analyzer, specifically "Multisizer™ 4" commercially available from by Beckman Coulter, Inc. The measurement may be preferably conducted at the room temperature (15 to 30 degrees C).

In view of providing improved storage stability of the vesicle composition at higher temperature, the vesicle composition obtainable by the preparation method of the present embodiment may preferably further contain a component (E): fatty acid ester. In addition, it is preferable that the component (E) is added in the oil phase during the oil phase-preparation step to obtain the vesicle composition containing the component (E) by the preparation method described above.

One, two or more selected from stearyl stearate, hydrogenated jojoba oils such as extremely hydrogenated jojoba oil and the like, myristyl myristate, and cetyl palmitate may be preferable for the component (E), and stearyl stearate is more preferable.

The content of the component (E) may be preferably equal to or higher than 0.01 % by mass over the vesicle composition, and may be more preferably equal to or higher than 0.05 % by mass. On the other hand, the content of the component (E) may be preferably equal to or lower than 3.0 % by mass over the vesicle composition, and may be more preferably equal to or lower than 2.0 % by mass.

Also, an arbitrary component may be added to the oil phase in the oil phase-preparation step without obstructing the production of the vesicle of the present embodiment. One, two or more selected from, for example, various types of extracts and antioxidants and the like may be employed for such arbitrary component, though it is not limited thereto. The arbitrary component, which is able to be added to the oil phase, is for example equal to or lower than 1 % mass of the whole oil phase, in view of the stable production of the vesicle composition.

In addition, an arbitrary component may be additionally contained in the aqueous phase in the emulsification step without obstructing the production of the vesicle of the present embodiment. Typical arbitrary component, which can be added thereto, includes, for example, one, two or more selected from various types of extracts and antiseptic agents and the like, though it is not limited thereto. The arbitrary component, which is able to be added to the aqueous phase, is for example equal to or lower than 0.1 % mass of the whole aqueous phase, in view of the stable production of the vesicle composition.

In the next, the hair cosmetic composition employing the vesicle composition obtainable in the present embodiment will be described.

The hair cosmetic composition in the present embodiment contains, for example, one of or a plurality of surfactants and an aliphatic alcohol, and further contains the above-described vesicle composition.

The content of the vesicle composition in the hair cosmetic composition may be established so as to provide the content of the component (A) constituting the vesicle of 0.01 to 5 % by mass and more preferably 0.05 to 2 % by mass, in view of providing improved spread feel in the application and the smoothness in the rinsing. Such hair cosmetic composition stably provides enhanced spread feel in the application as compared with the conventional hair cosmetic composition, even if the contents of the active constituents of such hair cosmetic composition is substantially equivalent to that of the conventional hair cosmetic composition.

Also, the hair cosmetic composition contains water. It is preferable to employ pure water. The content of water is not limited, and may be suitably adjusted for the use according to the applications.

The hair cosmetic composition typically includes, for example, hair conditioners, rinses, hair treatments, shampoos and the like. Hair conditioners, rinses and hair treatments are preferable as further effective hair cosmetic compositions. Further specifically, the hair cosmetic composition may be in the forms of hair treatments, conditioners or rinses, and may be a product obtainable by adding the vesicle composition of the present embodiment in a hair cosmetic composition base, which contains one, two or more surfactant(s) and an aliphatic alcohol, or preferably contains one, two or more surfactant(s), an aliphatic alcohol and emulsified silicone particles. These hair cosmetic compositions may be either in the type of usage for being washed away or in the type of usage for being not washed away after the hair cosmetic composition is applied.

The method for producing the hair cosmetic composition containing the vesicle composition includes, for example,

(I) a hair cosmetic composition base-preparation step; and
(II) a vesicle blending step for adding or blending the vesicle composition obtained in the hair cosmetic composition base-preparation step in the composition (the hair cosmetic composition base) obtained in (I).

Here, the hair cosmetic composition base is a generally employed composition, which contains one, two or more of the respective surfactant(s) and aliphatic alcohol(s), and optionally contains silicones such as emulsified silicone particle and the like or an oily component as required.

First of all, (I) the hair cosmetic composition base-preparation step is not limited to any specific process step, and may be conducted according to a process step for ordinarily producing the hair cosmetic composition. For example, an oil phase containing a cationic surfactant and a higher alcohol is added to an aqueous phase that is heated and stirred to achieve the emulsification to obtain the hair cosmetic composition base. In view of providing the storage stability of the obtainable vesicle composition, the smoothness in the rinsing and the smoothness after the drying, the temperature in the emulsification during the production of the hair cosmetic composition base may be preferably equal to or lower than the gel transition temperature of the obtainable hair cosmetic composition base.

Also, (II) the vesicle blending step for blending the vesicle composition obtained in the present embodiment to the hair cosmetic composition base obtained in (I) is not limited to any specific process step, as long as the step is related to blending the vesicle composition in the hair cosmetic composition base. From the viewpoint of the stability of the vesicle, the temperature during the addition of the vesicle composition may be preferably equal to or lower than the gel transition temperature of the vesicle. This allows obtaining the hair cosmetic composition, which retains the structure of the vesicle composition.

One of the constitution of first embodiment and the constitutions of the following embodiments may be carried out alone, or any combination thereof may be employed.

(Second Embodiment)

The above-described Patent Document 2 did not address the range of the production conditions related to the structural formation of the vesicle. Then, the present inventors studied the production conditions in detail, and found that the selection of the operating conditions in the phase inversion emulsification, the shapes of the blending vessel and the impeller, and the total quantity of the oil phase and the aqueous phase loaded in the blending vessel (hereinafter referred to as "total loading quantity") may create other structures except the vesicle structure, which may, in turn, reduce the smoothness from the rinsing through the post drying.

In addition, while Patent Document 3 discloses that enhanced emulsified liquid is obtained by suppressing the moisture adsorption to the water-soluble polymer, the disclosure is not related to achieving the spread feel in the application of the hair cosmetic composition and obtaining the smoothness in the rinsing.

The present embodiment provides the preparation method which can form vesicle by high performance.

The present inventors studied the methods for producing the vesicles by carrying out the phase inversion emulsification with a combination of a specific organic acid, a specific tertiary amine and a specific fatty acid, which were

stirred during the emulsification, and found that the phase inversion emulsification conducted under the conditions that the maximum of the power consumption falls within a specific range achieves suppressed formation of the structures except the vesicles to provide the effective creation of the vesicles, and further, the hair cosmetic composition containing the obtained vesicle composition exhibited enhanced smoothness from the rinsing through the post drying while suppressing the slimy feel in the rinsing and the pastiness after the drying.

According to the present embodiment, there is provided a method for producing a vesicle composition having an aqueous phase as a continuous phase, including:

(i) a step (an oil phase-preparation step) for dissolving components (A) to (C) at a temperature of equal to or higher than melting point of the oil phase, the aforementioned components (A) to (C) being:

the component (A): a fatty acid of total carbon number of from 12 to 40;
the component (B): a tertiary amine compound of total carbon number of from 8 to 75; and
the component (C): oil phase containing organic acid of total carbon number of from 1 to 10, and

(ii) a step (an emulsification step) for adding an aqueous phase in the oil phase obtained in the aforementioned step (i) while being mixed, in which the aforementioned mixing is carried out in the step (ii) (the emulsification step) so that the maximum value of power consumption Pv is equal to or larger than 0.1 $kW/m^3$ and equal to or smaller than 10 $kW/m^3$.

According to the present embodiment, the vesicle exhibiting enhanced smoothness from the rinsing through the post drying can be produced with enhanced efficiency while suppressing the slimy feel in the rinsing and the pastiness after the drying.

The method for producing the vesicle in the present embodiment includes the following steps (i) and (ii).

(i) (oil phase-preparation step) the step for dissolving components (A) to (C) at a temperature of equal to or higher than melting point of the oil phase, the aforementioned components (A) to (C) being:

the component (A): a fatty acid of total carbon number of from 12 to 40;
the component (B): a tertiary amine compound of total carbon number of from 8 to 75; and
the component (C): oil phase containing organic acid of total carbon number of from 1 to 10, and

(ii) (emulsification step) the step for adding an aqueous phase in the oil phase obtained in the aforementioned step (i) while being mixed.

Then, the mixing is carried out in the step (ii) so that the maximum value of power consumption Pv is equal to or larger than 0.1 $kW/m^3$ and equal to or smaller than 10 $kW/m^3$ to obtain the vesicle composition containing the continuous phase composed of the aqueous phase. The respective steps and the respective components will be specifically described below.

In the beginning, the components (A) to (C) will be described. In the present embodiment, the compounds as described in First Embodiment may be employed as the component (A).

Among the compounds for the component (A), preferable examples of the linear fatty acid represented by General formula (1) specifically include stearic acid, myristic acid, behenic acid and the like.

Branched fatty acid represented by General formula (2) may be of total carbon number of from 12 to 40, and preferably of from 8 to 30, and more preferably of from 10 to 22. In addition, the total carbon number may be preferably from 16 to 22.

Respective two or more types of the fatty acids represented by General formula (1) or (2) may be employed for the component (A), or two or more types of the fatty acids represented by different general formulae may alternatively be jointly employed.

It is preferable that the content of the component (A) falls within the range described in First Embodiment.

Next, the component (B) will be described. In the present embodiment, the compounds described in First Embodiment may be also employed as the component (B).

The component (B) is a tertiary amine compound of total carbon number of from 8 to 75, and more specifically is tertiary amine compound(s) represented by General formulae (3), (4) or (5).

In relation to tertiary amine compounds of the component (B) represented by General formula (3) and General formula (4), $R^2$ preferably represents linear or branched alkyl group or alkenyl group having carbon number of from 12 to 24, more preferably represents linear or branched alkyl group or alkenyl group of carbon number of from 14 to 24, and further preferably represents linear alkyl group of carbon number of from 18 to 22 in these formulae.

As the tertiary amine compounds of the component (B) represented by General formula (5), $R^5$ preferably represents linear or branched alkyl group or alkenyl group having carbon number of from 13 to 23, and more preferably represents linear or branched alkyl group or alkenyl group of carbon number of from 17 to 23.

Further, in the present embodiment, the tertiary amine compounds represented by General formula (6) described in First Embodiment may be employed for the component (B).

One, two or more of the tertiary amine(s) of the component (B) may be used together. Typical tertiary amine of the component (B) may be preferably ether amine of general formula (3) or amido amine of general formula (5).

The content of the component (B) may be preferably falls within the range described in First Embodiment, in view of providing the smoothness in the rinsing and the smoothness in the drying.

In the next, the component (C) will be described.

The component (C) is an organic acid having total carbon number of from 1 to 10. In the present embodiment, the compounds described in First Embodiment may be also employed as the component (C).

It is preferable that the content of the component (C) falls within the range described in First Embodiment.

The above-described compounds can be suitably combined to provide a suitable combination of the component (A), the component (B), and the component (C). Although it is not intended to limit the specific combination, in the case that the component (B) is N,N-dimethyl-3-octadecyloxy propyl amine and the component (C) is lactic acid, the component (A) may be preferably 18-methyl eicosanic acid, stearic acid, isostearic acid and the mixture thereof.

The component (A), the component (B) and the component (C) and water creates the vesicle, and more specifically, the vesicle composition containing multiple-layered lamella vesicle (so-called onion-vesicle) constituted of several double membranes dispersed in water are easy to be created. In the mean time, while the vesicle ordinarily means coacervates having hollow or water-phase internal layer, the multiple-layered lamella vesicle created here also generally includes coacervates having the constitution, in which a part of or the entire of the internal layer is oil phase. Further, in the present application, the "vesicle" also includes the multiple-layered lamella vesicle.

In view of providing increased volumetric concentration of the vesicle in the vesicle dispersion solution, the molar ratio (A)/(C) of the component (A) over the component (C) may be preferably within the range as described in First Embodiment.

Also, in view of achieving effective contribution of the component (A), the component (B) and the component (C) for creating the vesicle, ((A)+(C))/(B) may be preferably within the range as described in First Embodiment.

Further, in view of enhancing the storage stability and the handling ability of the vesicle dispersion solution, the sum of the component (A), the component (B) and the component (C) in the vesicle dispersion or the vesicle composition is preferably within the range as described in First Embodiment.

In addition, the mean particle diameter of the vesicle contained in the vesicle composition may be, in view of providing further improved spread feel when being applied over the hair, for example equal to or larger than 2 μm, preferably equal to or larger than 3 μm, and further preferably equal to or larger than 5 μm, and on the other hand, may be for example equal to or smaller than 20 μm, preferably equal to or smaller than 18 μm, and further preferably equal to or smaller than 15 μm. Here, the mean particle diameter may be measured as the volumetric mean particle diameter by employing, for example, a particle size analyzer, specifically "Multisizer™ 4" manufactured by Beckman Coulter, Inc. The measurement may be preferably conducted at the room temperature (15 to 30 degrees C).

In the next, the respective steps in the method for producing the vesicle composition will be further specifically described.

It is desirable that the vesicle composition of the present embodiment is constituted in the form of a dispersion solution (premix) of the vesicle. Such vesicle dispersion solution may be preferably produced by conducting, for example, the following process steps, which include:

(i) the process step for dissolving the oil phase containing the component (A), the component (B) and the component (C) at a temperature of equal to or higher than the melting point of the oil phase, and
(ii) the process step for adding an aqueous phase to the oil phase obtained in the above-described process step (i) while being mixed. According to such a procedure, the vesicle composition containing the continuous phase composed of the aqueous phase is obtainable.

The step (i) is the oil phase-preparation step. First of all, in the step (i), it is necessary to dissolve the oil phase, in view of achieving the stable production. For this reason, it is preferable to achieve the dissolution at a temperature that is equal to or higher than the melting point of the oil phase, and is further preferable to achieve the dissolution at a temperature that is equal to or higher by 5 degrees C than the melting point of the oil phase, and is more preferable to achieve the dissolution at a temperature that is equal to or higher by 10 degrees C than the melting point of the oil phase.

Also, it is preferable that the oil phase is in the condition of being uniformly mixed. Thus, it is preferable to carry out the dissolution in the present step while mixing the oil phase. While the mixing process is not limited, it is preferable to mix by, for example, stirring.

In the next, in the process step (ii), an aqueous phase is dripped in the oil phase dissolved in the process step (i), which is more specifically the oil phase of the shear-mixed condition. The step (ii) is the emulsification step. While the available mixing equipment is not limited as long as the shear-mixing can be achieved, if the mixture exhibits higher viscosity in the process of adding the aqueous phase, it is preferable to employ an apparatus which is applicable for mixing the high viscosity product, such as, for example: AGI HOMO MIXER and TK COMBI MIX, commercially available from PRIMIX Corporation; Vacuum Emulsification Stirring Apparatus, commercially available from MIZUHO Industrial Co., Ltd.; Maxblend, commercially available from Sumitomo Heavy Industries, Ltd.; Super Mix Stirring Vessel commercially available from Satake Chemical Equipment Mfg Ltd., and the like.

Also, the mixing may be carried out in the step (ii) such that the maximum value of the power consumption Pv falls within the above-described specific range. This allows suppressing coexisting with the layered lamella or the like to achieve effective formation of the vesicle structure and providing stable improvement in the performances of the hair cosmetic composition obtainable by using the vesicle composition.

Meanwhile, Pv is an indicator for evaluating the degree of mixing of the liquid in the blending vessel, which is determined generally by geometric factors of the a blending vessel and the impeller, rotational speed of the impeller, density, viscosity and volume of the liquid in the blending vessel and the like.

In the present embodiment, the maximum value of Pv in the step (ii) is established as being equal to or larger than 0.1 $kW/m^3$. This allows providing improved degree of mixing of the oil phase and the aqueous phase and providing the preferable mixing under the condition of the balancing between the vesicle volumetric concentration (volumetric ratio of the vesicle occupied in the dispersion solution) and the viscosity of the dispersion solution, so that a formation of the layered lamella is suppressed to obtain the vesicle structure with improved efficiency. In view further effectively producing the vesicle, the maximum value of Pv in the step (ii) may be preferably equal to or higher than 0.5 $kW/m^3$, more preferably equal to or higher than 0.8 $kW/m^3$, and further preferably equal to or higher than 1.5 $kW/m^3$.

On the other hand, the maximum value of Pv is established as being equal to or lower than 10 $kW/m^3$, so that the smoothness can be more stably improved from the rinsing through the post drying, while suppressing the slimy feel in the rinsing and the pastiness after the drying, in the case that the vesicle composition is blended in the hair cosmetic composition. It is considered that the reason for this is related to the fact that excessively larger maximum value of Pv provides excessively reduced water content in the vesicle. In view of providing further improved smoothness of the hair cosmetic composition containing the vesicle composition blended therein from the rinsing through the post drying and providing further improved stability in the industrial production of the vesicle, the maximum value of Pv in the step (ii) may be preferably equal to or lower than 7 $kW/m^3$, more preferably equal to or lower than 5 $kW/m^3$, and further preferably equal to or lower than 3 $kW/m^3$.

In the step (ii), the factors for controlling the maximum value of Pv may include the geometric factors of the a blending vessel and the impeller, the rotational speed of the impeller, the time for dripping the aqueous phase, and the density, the viscosity and the volume of the liquid in the blending vessel and the like. Among these, in view of facilitating the control of the maximum value of the Pv, it is preferable to control the maximum value of Pv by suitably controlling the rotational speed of the impeller, the time for dripping the aqueous phase. The maximum value of Pv may be increased by, for example, increasing the rotational speed of the impeller. It is preferable that the rotational speed of impeller is adjusted to, for example, from 15 to 800 rpm.

In addition, the maximum value of Pv can also be increased by increasing the time for dripping the aqueous phase. The time for dripping may be for example equal to or longer than 5 minutes, and preferably equal to or longer than 20 minutes, and on the other hand, may be equal to or shorter than 100 minutes, and preferably equal to or shorter than 80 minutes.

The degree of mixing of the oil phase and the aqueous phase is improved to increase the maximum value of Pv by increasing the rotational speed (number of rotations) of the impeller or by increasing the time for dripping the aqueous phase, so that the efficiency for creating the vesicle structure is increased. On the other hand, excessively higher rotational speed (number of rotations) of the impeller or excessively longer time for dripping the aqueous phase causes excessively reduced water content in the vesicle, which may cause insufficient exhibition of the performances of the hair cosmetic composition. For this reason, the mixing condition is determined in the step (ii) so that the maximum value of Pv falls within the specific range described above.

In the step (ii), Pv can be directly measured by employing, for example, a clamp-type power meter commercially available from HIOKI E.E. CORPORATION or the like. In view of easily calculating the maximum Pv value, it is preferable that the power is actually measured.

When Pv is directly measured, it can be calculated by dividing the net stirring power, which is obtainable by subtracting the stirring power in the idle-running from the directly-measured stirring power, by the quantity of the solution contained in the blending vessel at that time, as indicated by the following equation (II).

$$Pv = (Pi-P0)/V \ldots (II)$$

(In the above-described equation (II), Pi is the directly-measured stirring power [kW] and P0 is the stirring power in the idle-running [kW]. In addition, V is the volume of the liquid in the blending vessel [$m^3$].)

Concerning the control of the structure by selecting the value of Pv, larger total loading quantity in the blending vessel provides more precise control. In addition, concerning the control of the value of Pv itself, larger total loading quantity in the blending vessel provides the control with more enhanced precision.

Thus, in view of providing improved controllability of the maximum value of Pv, it is preferable to conduct the phase inversion emulsification by carrying out the mixing under the condition that the total weight of the oil phase and the aqueous phase for loading in the blending vessel is equal to or larger than 10 kg, and more preferably equal to or larger than 100 kg, and further preferably equal to or larger than 200 kg.

Further, the particle diameter of the vesicle may be controlled by suitable controlling a stirring rotational speed after the start of the dripping of the aqueous phase. The stirring rotational speed after the start of the dripping of the aqueous phase may be preferably 15 to 800 rpm, in view of obtaining the vesicle having the particle diameter, which provides further improved smoothness from the rinsing through the post drying while suppressing the slimy feel in the rinsing and the pastiness after the drying. More specifically, the stirring rotational speed after the start of the dripping of the aqueous phase is preferably equal to or higher than 15 rpm, and is preferably equal to or lower than 800 rpm. Also, in the present embodiment, the stirring rotational speed may alternatively be as disclosed in First Embodiment.

Also, in view of stably providing improved smoothness from the rinsing through the post drying, while suppressing the slimy feel in the rinsing and the pastiness after the drying in the hair cosmetic composition containing the vesicle composition blended therein, the volumetric concentration of the vesicle in the vesicle dispersion solution may be preferably higher. The volumetric concentration of the vesicle in the vesicle dispersion solution can be adjusted by adjusting the dripping rate of the aqueous phase to the oil phase and the stirring rotational speed in dripping the aqueous phase. The optimum values of the dripping rate of the aqueous phase to the oil phase and the stirring rotational speed in the dripping can be suitably changed with the formulation and the component ratio of the vesicle composition and the size and of the shape of the blending vessel, and it is preferable to employ the condition that can achieve the uniform mixing even in the state of the highest viscosity during the aqueous phase dripping. Further continuation of the dripping of the aqueous phase causes decrease in the viscosity of the vesicle dispersion solution to provide reduced volumetric concentration of the vesicle. The quantity of the dripping aqueous phase can be suitably adjusted in consideration of the storage stability and the handling ability of the vesicle dispersion solution.

The dripping rate of the aqueous phase to the oil phase can be suitably selected as described above, and for enhancing the volumetric concentration of the vesicle in the vesicle dispersion solution, it is preferable to take equal to or longer than 5 minutes to carry out the dripping, though it is not intended to limit the dripping rate.

While the temperature in the aqueous phase dripping can be suitably determined in the step (ii) by the temperature of the oil phase and the temperature of the aqueous phase to be dripped and the heating or cooling in the mixing equipment, it is preferable to select the temperature of the oil phase and the temperature of the aqueous phase to be dripped so as to be higher than the gel transition temperature of the vesicle to be created. As described above, the procedure for the emulsification, in which the aqueous phase is dripped while stirring the oil phase, is generally referred to as a phase inversion emulsification. In the present embodiment, the oil phase is constituted as described above to create the vesicle composition by the phase inversion emulsification, such that the vesicle composition acquires enhanced handling ability.

While pure water such as deionized water, distilled water or the like may be employed for the "aqueous phase", a polyol, which is the component (D) soluble in water, more specifically for example glycerol, dipropylene glycol and the like, may be additionally contained, as will be discussed later.

Also, in view of providing the stability of the obtainable vesicle composition, it is preferable to be the method for producing the vesicle composition including a step (iii) for rapidly cooling thereof to a temperature of equal to or lower than the gel transition temperature of the vesicle after the step (ii) of dripping the aqueous phase is finished. The step (iii) is the cooling step.

In the present embodiment, the oil phase may further contain the component (D): polyol (first polyol) in the method for producing the vesicle composition.

Also, the vesicle composition obtainable by the production method of the present embodiment may further contain (D) a polyol.

Typical component (D) may include, more specifically, ethylene glycol, diethylene glycol, propylene glycol, dipropylene glycol, glycerol and the like. Among these, propylene glycol and dipropylene glycol are preferable.

The content of the component (D) may be preferably 0.5 to 60 % by mass over the whole vesicle composition in view of the storage stability of the vesicle composition, and more preferably 1 to 50 % by mass. It is further preferable

to be 2 to 20 % by mass.

While the addition of the component (D) in the vesicle composition may be carried out by adding thereof in the aqueous phase in the step (ii) as described above, it is preferable to add in the oil phase in the step (i). It is preferable to add the component (D) in the oil phase in the step (i) after the oil phase containing the component (A), the component (B) and the component (C) is dissolved at a temperature of equal to or higher than the melting point of the oil phase. Alternatively, the oil phase may be obtained in the step (i), so that the oil phase containing the component (A), the component (B), the component (C) and the component (D) is dissolved at a temperature of equal to or larger than the melting point of the oil phase until a solid matter is disappears. After the step (i), the step (ii) of adding the aqueous phase to the obtained oil phase while mixing thereof is conducted to obtain the vesicle dispersion solution having higher vesicle volumetric concentration and further higher storage stability. Further, from the viewpoint of the storage stability of the vesicle, it is preferable to carry out the step (iii) for cooling thereof to a temperature of equal to or lower than the gel transition temperature of the vesicle, rapidly after the aqueous phase dripping is completed.

Alternatively, an arbitrary component may be added in the oil phase in the step (i) as long as the production for the vesicle of the present embodiment is not obstructed. Such arbitrary component may include, for example, various types of extracts and antioxidants and the like, though it is not limited thereto. The arbitrary component, which is able to be added to the oil phase, is for example equal to or lower than 1 % by mass of the whole oil phase, in view of the stable production of the vesicle composition.

Alternatively, an arbitrary component may be added in the aqueous phase in the step (ii) as long as the production for the vesicle of the present embodiment is not obstructed. Such arbitrary component available to be added may include, for example, various types of extracts and antiseptic agents and the like, though it is not limited thereto. The arbitrary component, which is able to be added to the aqueous phase, is for example equal to or lower than 0.1 % mass of the whole aqueous phase, in view of the stable production of the vesicle composition.

Concerning the higher alcohol, it is preferable for the formulation containing the component (A), the component (B) and the component (C) of the present embodiment to contain no or substantially no higher alcohol in the vesicle composition, in view of increasing the volumetric fraction of the vesicle contained in the composition, and it may contain thereof at equal to or lower than 2 % by mass in the vesicle composition, and may be further preferably equal to or lower than 1 % by mass.

Next, the hair cosmetic composition employing the vesicle composition obtained in the present embodiment will be described. The hair cosmetic composition in the present embodiment contains, for example, one of or a plurality of surfactants and an aliphatic alcohol, and further contains the above-described vesicle composition.

The content of the vesicle composition in the hair cosmetic composition may be established so as to provide the content of the component (A) constituting the vesicle of 0.01 to 5 % by mass and more preferably 0.05 to 2 % by mass, in view of providing improved spread feel in the application and the smoothness in the rinsing. Such hair cosmetic composition stably provides enhanced spread feel in the application as compared with the conventional hair cosmetic composition, even if the contents of the active constituents of such hair cosmetic composition is substantially equivalent to that of the conventional hair cosmetic composition.

Such hair cosmetic composition typically includes, for example, hair conditioners, rinses, hair treatments, shampoos and the like. Hair conditioners, rinses and hair treatments are preferable as further effective hair cosmetic compositions. These hair cosmetic compositions may be either in the type of usage for being washed away or in the type of usage for being not washed away after the hair cosmetic composition is applied.

The hair cosmetic composition containing the vesicle composition is obtainable by mixing the vesicle composition of the present embodiment to a hair cosmetic composition base, which is separately prepared by an ordinary method. This hair cosmetic composition base prepared by the ordinary method is a general hair cosmetic composition containing, for example, a surfactant and an aliphatic alcohol, and additionally containing silicone, an oily ingredient or the like as required. This can be prepared by an arbitrary method.

Although the formulation and the production method of the hair cosmetic composition base are not limited, the hair cosmetic composition base is obtainable by, for example, adding the oil phase containing cationic surfactant and higher alcohol to the heated and stirred aqueous phase to cause the emulsification.

The hair cosmetic composition is further specifically in conformations of a conditioner or a rinse that may be obtainable by adding the vesicle composition to a base treatment, a base conditioner or a base rinse, which contains one or a plurality of surfactant(s), an aliphatic alcohol and emulsified silicone particles.

While the type of the method for blending the vesicle composition in the ordinary hair cosmetic composition base is not limited, it is preferable to blend at a temperature of equal to or lower than the gel transition temperature of the vesicle, in view of the stability of the vesicle. This allows obtaining the hair cosmetic composition, which retains the structure of the vesicle composition.

Concerning the embodiments described above, The present invention will further disclose the following compositions, production methods, and the applications.

<1> A method for producing a vesicle composition having an aqueous phase as a continuous phase, including:

an oil phase-preparation step for dissolving components (A) to (D) at a temperature of equal to or higher than melting point of the oil phase, the aforementioned components (A) to (D) being:

the component (A): a fatty acid of total carbon number of from 12 to 40;
the component (B): a tertiary amine compound of total carbon number of from 8 to 75;
the component (C): an organic acid of total carbon number of from 1 to 10; and
the component (D): the first polyol,

an emulsification step for adding the aqueous phase to the oil phase obtained in the oil phase-preparation step while being mixed;
an addition step for adding a component (D'): the second polyol, after a quantity of the aqueous phase added to the oil phase is equal to or larger than 1 time by mass of a quantity of the oil phase; and
a cooling step for cooling the obtained composition after the emulsification step.

<2> The method for producing the vesicle composition according to <1>, wherein the aforementioned mixing is carried out preferably in the aforementioned emulsification step so that the maximum value of the power consumption Pv is equal to or higher than 0.1 $kW/m^3$, preferably equal to or higher than 0.5 $kW/m^3$ and more preferably equal to or higher than 0.8 $kW/m^3$, and further preferably equal to or higher than 1.5 $kW/m^3$, and on the other hand, is equal to or lower than 10 $kW/m^3$, preferably equal to or lower than 7 $kW/m^3$, more preferably equal to or lower than 5 $kW/m^3$, and even more preferably equal to or lower than 3 $kW/m^3$.
<3> The method for producing the vesicle composition according to <1> or <2>, wherein the phase inversion emulsification is preferably conducted by carrying out the aforementioned mixing under the condition that the total weight of the oil phase and the aqueous phase for loading in the blending vessel is equal to or larger than 10 kg, more preferably equal to or larger than 100 kg, and further preferably equal to or higher than 200 kg.
<4> The method for producing the vesicle composition according to any one of <1> to <3>, wherein the addition step is preferably conducted after the emulsification step, and further preferably conducted after the cooling step.
<5> The method for producing the vesicle composition according to any one of <1> to <4>, wherein the temperature in the mixing in the emulsification step is preferably equal to or higher than the gel transition temperature of the obtained vesicle composition.
<6> The method for producing the vesicle composition according to any one of <1> to <5>, wherein the vesicle composition is preferably cooled to equal to or lower than the gel transition temperature in the cooling step.
<7> The method for producing the vesicle composition according to any one of <1> to <6>, wherein the temperature for dissolving the oil phase in the oil phase-preparation step is preferably a temperature that is equal to or higher by 5 degrees C than the melting point of the oil phase, and more preferably a temperature that is equal to or higher by 10 degrees C.
<8> The method for producing the vesicle composition according to any one of <1> to <7>, wherein the oil phase-preparation step preferably includes adding the component (E): fatty acid ester in the oil phase, and preferably adding one, two or more selected from stearyl stearate, hydrogenated jojoba oil, myristyl myristate and cetyl palmitate, and further preferably adding stearyl stearate.
<9> The method for producing the vesicle composition according to any one of <1> to <8>, wherein the component (D') is preferably one, two or more compound(s) selected from ethylene glycol, diethylene glycol, propylene glycol, dipropylene glycol, glycerol and the like, and is more preferably one, two or more compound(s) selected from propylene glycol and dipropylene glycol, and further preferably is propylene glycol and dipropylene glycol.
<10> The method for producing the vesicle composition according to any one of <1> to <9>, wherein the content of the component (D') is preferably equal to or higher than 1 % by mass in the vesicle composition, and more preferably equal to or higher than 3 % by mass, and further preferably equal to or higher than 5 % by mass, and on the other hand, is equal to or lower than 30 % by mass, and preferably equal to or lower than 20 % by mass, and further preferably equal to or lower than 15 % by mass; or wherein the adding quantity of the component (D') is from 1 to 30 % by mass in the vesicle composition.
<11> The method for producing the vesicle composition according to any one of <1> to <10>, wherein, when the component (D') is preferably propylene glycol and dipropylene glycol, ratio of dipropylene glycol and propylene glycol in the component (D') is from 10:90 to 90:10, and preferably at from 20:80 to 80:20.
<12> The method for producing the vesicle composition according to any one of <1> to <11>, wherein preferably the quantity of the aqueous phase added in the emulsification step is equal to or higher than 1 time of the oil phase by mass, is preferably equal to or higher than twice of the oil phase by mass thereof, and is further preferably equal to or higher than 3 times by mass thereof, and on the other hand, is equal to or lower than 100 times of the oil phase

by mass, and is more preferably equal to or lower than 50 times of the oil phase by mass.

<13> The method for producing the vesicle composition according to any one of <1> to <12>, wherein preferably the content of the component (A) is equal to or higher than 0.1 % by mass in the vesicle composition and preferably equal to or higher than 1 % by mass, and on the other hand is equal to or lower than 10 % by mass and preferably equal to or lower than 5 % by mass;

the content of the component (B) is equal to or higher than 0.5 % by mass in the vesicle composition, preferably equal to or higher than 1 % by mass and more preferably equal to or higher than 1.5 % by mass, and on the other hand is equal to or lower than 15 % by mass, preferably equal to or lower than 10 % by mass and more preferably equal to or lower than 7 % by mass;

the content of the component (C) is equal to or higher than 0.05 % by mass in the vesicle composition and preferably equal to or higher than 0.1 % by mass, and on the other hand is equal to or lower than 4 % by mass and preferably equal to or lower than 2 % by mass; and

the content of the component (D) is equal to or higher than 0.5 % by mass in the vesicle composition, preferably equal to or higher than 1 % by mass and more preferably equal to or higher than 2 % by mass, and on the other hand is equal to or lower than 40 % by mass, preferably equal to or lower than 30 % by mass and more preferably equal to or lower than 20 % by mass; or in the oil phase-preparation step, the adding quantity of the component (D) is from 0.5 to 40 % by mass in the vesicle composition. Or, the method for producing the vesicle composition according to any one of <1> to <12>, wherein, in the production method of the above-described <13>, the adding quantity of the component (D) is equal to or higher than 0.5 % by mass and equal to or lower than 40 % by mass in the vesicle composition in the oil phase-preparation step.

<14> The method for producing the vesicle composition according to any one of <1> to <13>, wherein preferably the total carbon number of the component (A) is equal to or higher than 14 and preferably equal to or larger than 16, and on the other hand is equal to or lower than 30, preferably equal to or lower than 24, and more preferably equal to or lower than 22.

<15> The method for producing the vesicle composition according to any one of <1> to <14>, wherein preferably the component (A) is one, two or more selected from linear fatty acids represented by the above-described general formula (1) or branched saturated fatty acids represented by the above-described general formula (2), and is more preferably a combination of a linear fatty acid represented by the above-described general formula (1) and a branched fatty acid represented by the above-described general formula (2).

<16> The method for producing the vesicle composition according to any one of <1> to <15>, wherein preferably the linear fatty acid represented by the above-described general formula (1) is one, two or more selected from palmitic acid, stearic acid, arachidic acid and behenic acid, and the branched fatty acid represented by the above-described general formula (2) is one, two or more selected from 2-heptyl undecanoic acid (isostearic acid), 14-methyl pentadecanoic acid, 15-methyl hexadecanoic acid, 16-methyl heptadecanoic acid, 17-methyl octadecanoic acid, 18-methyl nonadecane acid, 19-methyl eicosanic acid, 20-methyl heneicosanic acid, 14-methyl hexadecanoic acid, 15-methyl heptadecanoic acid, 16-methyl octadecanoic acid, 17-methyl nonadecane acid, 18-methyl eicosanic acid and 19-methyl heneicosanic acid.

<17> The method for producing the vesicle composition according to any one of <1> to <16>, wherein preferably, when the component (A) is a combination of a linear fatty acid represented by the above-described general formula (1) and a branched fatty acid represented by the above-described general formula (2), ratio of the linear fatty acid and the branched fatty acid is at the mass ratio of: (1)/(2) = 70/30 - 40/60.

<18> The method for producing the vesicle composition according to any one of <1> to <17>, wherein preferably the component (B) is one, two or more selected from amido amines, ether amines, alkylamines and hydroxyether amines, preferably from amido amine and ether amines, and more preferably from ether amines.

<19> The method for producing the vesicle composition according to any one of <1> to <18>, wherein preferably the component (C) is an organic acid of total carbon number of equal to or larger than 1 and preferably equal to or larger than 2, and on the other hand of equal to or smaller than 10 and preferably equal to or smaller than 8 and more preferably equal to or smaller than 6.

<20> The method for producing the vesicle composition according to any one of <1> to <19>, wherein preferably the component (C) is one, two or more compounds selected from monocarboxylic acids, dicarboxylic acids, hydroxy carboxylic acids, aromatic carboxylic acids, and acidic amino acids; and preferably selected from acetic acid, propionic acid, caprylic acid, capric acid, malonic acid, succinic acid, glutaric acid, adipic acid, maleic acid, fumaric acid, glycolic acid, lactic acid, hydroxy acrylic acid, glyceric acid, malic acid, tartaric acid, citric acid, benzoic acid, salicylic acid, phthalic acid, glutamic acid and asparagic acid.

<21> The method for producing the vesicle composition according to any one of <1> to <20>, wherein preferably in the components (A), (B) and (C), ratio of the sum of the acid equivalents of the component (A) and the component (C) over the base equivalent of the component (B) is equal to or higher than 0.25, and is preferably equal to or higher than 0.5, and is more preferably equal to or higher than 0.6, and on the other hand is equal to or lower than

4, preferably equal to or lower than 2 and more preferably equal to or lower than 1.8.
<22> The method for producing the vesicle composition according to any one of <1> to <21>, wherein preferably the molar ratio (A)/(C) of the component (A) and the component (C) is equal to or higher than 5/5 and preferably equal to or higher than 7/3, and on the other hand is equal to or lower than 9/1 and preferably equal to or lower than 8/2.
<23> The method for producing the vesicle composition according to any one of <1> to <22>, wherein preferably the sum of the component (A), the component (B) and the component (C) in the vesicle composition is equal to or higher than 1 % by mass and on the other hand is equal to or lower than 20 % by mass further preferably equal to or lower than 15 % by mass.
<24> The method for producing the vesicle composition according to any one of <1> to <23>, wherein preferably the rotational speed in the emulsification is from 15 to 800 rpm, or, the rotational speed in the emulsification is for example is equal to or higher than 15 rpm and preferably equal to or higher than 20 rpm, and on the other hand is for example equal to or lower than 800 rpm, preferably equal to or lower than 500 rpm and further preferably equal to or lower than 200 rpm.
<25> The method for producing the vesicle composition according to any one of <1> to <24>, wherein preferably the time for dripping is equal to or longer than 5 minutes and equal to or shorter than 100 minutes.
<26> A vesicle composition, preferably obtainable by the production method according to any one of <1> to <25>.
<27> A hair cosmetic composition preferably in a form of a treatment, a conditioner or a rinse, which is obtainable by adding the vesicle composition described in <26> to a hair cosmetic composition base containing one, two or more surfactant(s) and an aliphatic alcohol, preferably to a hair cosmetic composition base containing one, two or more surfactant(s) and an aliphatic alcohol.
<28> A method for producing a hair cosmetic composition, preferably including: a hair cosmetic composition base-preparation step for adding an oil phase containing a surfactant and an aliphatic alcohol in an aqueous phase to carry out an emulsification; and a vesicle blending step for adding the vesicle composition according to <26> in the composition obtained in the hair cosmetic composition base-preparation step.
<29> The method for producing a hair cosmetic composition according to <28>, wherein preferably the emulsified temperature in the hair cosmetic composition base-preparation step is equal to or lower than the gel transition temperature of the composition obtainable in the hair cosmetic composition base-preparation step.
<30> The method for producing a hair cosmetic composition according to <28> or <29>, wherein preferably the vesicle blending step is conducted at a temperature of equal to or lower than the gel transition temperature of the vesicle composition.

Also, the present invention contains the following specific modes.

<A1> A method for producing a vesicle composition having an aqueous phase as a continuous phase, including:

(i) a step for dissolving components (A) to (C) at a temperature of equal to or higher than melting point of the oil phase, the aforementioned components (A) to (C) being:

the component (A): a fatty acid of total carbon number of from 12 to 40;
the component (B): a tertiary amine compound of total carbon number of from 8 to 75; and
the component (C): an organic acid of total carbon number of from 1 to 10, and

(ii) a step for adding an aqueous phase to the oil phase obtained in the aforementioned step (i) while being mixed, wherein a mixing is carried out in the aforementioned step (ii) such that the maximum value of a power consumption Pv is equal to or higher than 0.1 $kW/m^3$ and equal to or lower than 10 $kW/m^3$.

<A2> The method for producing the vesicle composition according to <A1>, wherein the aforementioned component (A) is any one, two or more of fatty acids represented by the following general formula (1) or general formula (2).

[General formula 1]

$$H_3C-(CH_2)_n-COOH \qquad (1)$$

(In the above-described general formula (1), n represents an integer number of from 10 to 38).

[General formula 2]

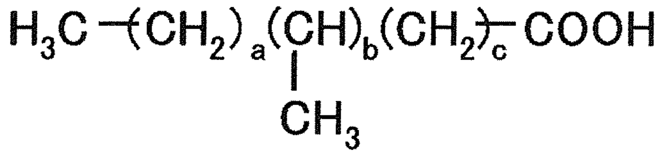

(2)

(In the above-described general formula (2), the sum of a, b and c is: a+b+c = 9 to 37; and b is 1).

<A3> The method for producing the vesicle composition according to <A1> or <A2>, wherein the aforementioned component (B) one or more of tertiary amine compounds selected from the group consisting of compounds represented by the following general formulae (3), (4) and (5),

[General formula 3]

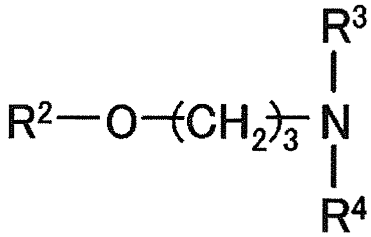

(3)

[General formula 4]

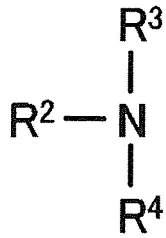

(4)

[General formula 5]

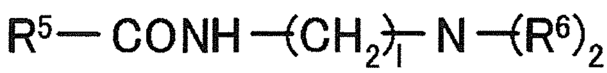

(5)

(In the above-described general formulae (3) and (4), $R^2$ represents linear or branched alkyl group or alkenyl group of carbon number of from 6 to 24, $R^4$ and $R^3$ are same or different alkyl group(s) of carbon number of 1 to 6 or $(AO)_mH$ (AO is oxy alkylene group of carbon number of 2 to 4, m represents an integer number of from 1 to 6, m of AO may be may the same or different and the sequence thereof is arbitrary). Also, in the above-described general formula (5), $R^5$ represents aliphatic hydrocarbon group of carbon number of from 11 to 23, $R^6$, each of which may be same or different, represents hydrogen atom or alkyl group of carbon number of from 1 to 4, and 1 represents an integer number of from 2 to 4.)

<A4> The method for producing the vesicle composition according to any one of <A1> to <A3>, wherein a phase inversion emulsification is achieved in the step (ii) by carrying out the mixing under the condition that the total weight of the oil phase and the aqueous phase for loading in the blending vessel is equal to or larger than 10 kg.

<A5> The method for producing the vesicle composition according to any one of <A1> to <A4>, wherein the aforementioned oil phase further contains the component (D): a polyol.

<A6> The method for producing the vesicle composition according to any one of <A1> to <A5>, wherein, among

the aforementioned components (A), (B) and (C), ratio of the sum of the acid equivalents of the component (A) and the component (C) over the base equivalent of the component (B) is from 0.25 to 4.
<A7> The method for producing the vesicle composition according to any one of <A1> to <A6>, wherein the sum of the aforementioned components (A), (B) and (C) in the vesicle composition is from 1 to 20 % by mass.
<A8> A vesicle composition produced by the method according to any one of <A1> to <A7>.
<A9> A hair cosmetic composition preferably in a form of a treatment, a conditioner or a rinse, which is obtainable by adding the vesicle composition described in <A8> to a base treatment, a base conditioner or a base rinse, which contain one or a plurality of surfactant(s), an aliphatic alcohol and emulsified silicone particles.
<B1> A method for producing a vesicle composition having an aqueous phase as a continuous phase, including:

an oil phase-preparation step for dissolving components (A) to (D) at a temperature of equal to or higher than melting point of the oil phase, the aforementioned components (A) to (D) being:

the component (A): a fatty acid of total carbon number of from 12 to 40;
the component (B): a tertiary amine compound of total carbon number of from 8 to 75;
the component (C): an organic acid of total carbon number of from 1 to 10; and
the component (D): an oil phase containing the first polyol,

an emulsification step for adding the aqueous phase to the oil phase obtained in the oil phase-preparation step while being mixed;
an addition step for adding a component (D'): the second polyol, after a quantity of the aqueous phase added to the oil phase is equal to or larger than 1 time by mass of a quantity of the oil phase; and
a cooling step for cooling the obtained composition after the emulsification step.

<B2> The method for producing the vesicle composition according to <B1>, wherein the adding quantity of the aforementioned component (D') is from 1 to 30 % by mass in the vesicle composition.
<B3> The method for producing the vesicle composition according to <B1> or <B2>, wherein the adding quantity of the aforementioned component (D) is from 0.5 to 40 % by mass in the vesicle composition.
<B4> The method for producing the vesicle composition according to any one of <B1> to <B3>, wherein the aforementioned oil phase further contains component (E): a fatty acid ester.
<B5> The method for producing the vesicle composition according to any one of <B1> to <B4>, wherein among the aforementioned components (A), (B) and (C), ratio of the sum of the acid equivalents of the component (A) and the component (C) over the base equivalent of the component (B) is from 0.25 to 4.
<B6> The method for producing the vesicle composition according to any one of <B1> to <B5>, wherein the sum of the aforementioned components (A), (B) and (C) in the vesicle composition is from 1 to 20 % by mass.
<B7> The method for producing the vesicle composition according to any one of <B1> to <B6>, wherein the addition step is conducted after the cooling step.
<B8> A vesicle composition produced by the method according to any one of <B1> to <B7>.
<B9> A hair cosmetic composition in a form of a treatment, a conditioner or a rinse, which is obtainable by adding the vesicle composition described in <B8> to a hair cosmetic composition base containing one, two or more surfactant(s) and an aliphatic alcohol.
<B10> A method for producing a hair cosmetic composition, including: a hair cosmetic composition base-preparation step for adding an oil phase containing a cationic surfactant and a higher alcohol to an aqueous phase to cause an emulsification; and a vesicle addition step for blending the vesicle composition according to <B8> in the composition obtained in the aforementioned hair cosmetic composition base-preparation step.

EXAMPLES

Embodiments of the present invention will be further specifically described in reference to the following Examples. Examples are for illustrating, and it is not intended to limit the scope of the present invention.

(Examples 1 to 15 and Comparative Examples 1 to 4)

Premixed compositions for the respective Examples were prepared according to the following methods. Also, the obtained premixed compositions were employed to conduct preparations and evaluations of rinses. Formulations of the premixes and the rinses, production conditions for the premixes, and the evaluation results of the rinse are shown in Table 1 to Table 3.

(Example 1)

The formulation of Table 1 was converted so that the total quantity of the premix is 1,800 g, and the respective components were weighed. The components (A), (B), (C), (D) and (E) were supplied to a separable flask of 2 L, and were heated to 80 degrees C while being stirred to completely dissolve the raw materials. A constant-quantity dripping of an aqueous phase of deionized water heated to 80 degrees C in this oil phase was carried out by spending 60 minutes to carry out the emulsification under the conditions of the temperature of 80 degrees C and a stirring rotational speed of 90 rpm. After the emulsification was completed, the resultant was cooled to 30 degrees C with a refrigerant of 5 degrees C. Thereafter, the component (D') was added, and then the solution was stirred at a stirring rotational speed of 90 rpm for five minutes to obtain a premixed composition.

In addition to above, ratio of the aqueous phase (W) and the oil phase (O) at the time of adding the second polyol was 6.49.

Production conditions for premixes and weight fractions of the respective components in the present Example and the following Examples are represented in Table 1 to Table 3.

(Example 2)

A premixed composition was obtained according to Example 1, except that the component (E) blended in the oil phase was changed from stearyl stearate to hydrogenated jojoba oil.

(Example 3)

A premixed composition was obtained according to Example 1, except that the blending quantity of the component (B) blended in the oil phase and the blending quantity of the deionized water were changed.

(Example 4)

A premixed composition was obtained according to Example 1, except that the component (E) was removed from the oil phase and the formulation of Table 1 was converted so that the total quantity of the premix is 1,800 g, and the respective components were weighed.

(Example 5)

A premixed composition was obtained according to Example 1, except that the component (D') was changed from dipropylene glycol into propylene glycol.

(Example 6)

A premixed composition was obtained according to Example 1, except that the blending quantity of the component (D') and the blending quantity of deionized water were changed.

(Example 7)

A premixed composition was obtained according to Example 1, except that the blending quantities of the component (D') and the deionized water were adjusted so that dipropylene glycol and propylene glycol in the component (D') were 1:1 (mass ratio).

(Example 8)

A premixed composition was obtained according to Example 7, except that the component (B) blended in the oil phase was changed to N-[3-(dimethylamino)propyl]docosane amide and that the quantities of the component (B) and the deionized water were adjusted so that ((A) + (C))/(B) was 1.0.

(Example 9)

A premixed composition was obtained according to Example 1, except that the components (A) of the oil phase were only stearic acid and isostearic acid, and that the formulation described in Table 2 except the component (E) were converted so that the total quantity of the premix is 1,800 g, and the respective components were weighed.

(Example 10)

A premixed composition was obtained according to Example 1, except that the component (A) of the oil phase was changed to only stearic acid, and that the formulation described in Table 2 related to the component (D'), which were determined as containing dipropylene glycol and propylene glycol at 1:1 (mass ratio), were converted so that the total quantity of the premix is 1,800 g, and the respective components were weighed.

(Example 11)

A premixed composition was obtained according to Example 1, except that the composition was cooled to 70 degrees C with a refrigerant of 5 degrees C after the emulsification was completed, and after that, dipropylene glycol of the component (D') was blended and was stirred.

(Example 12)

The formulation of Table 2 was converted so that the total quantity of the premix is 1,800 g, and the respective components were weighed. The components (A), (B), (C), (D) and (E) were supplied to a separable flask of 2 L, and were heated to 80 degrees C while being stirred to completely dissolve the raw materials. An aqueous phase of deionized water heated to 80 degrees C was carried out into this oil phase by spending 25 minutes until the ratio of aqueous phase (W) and oil phase (O) reaches 2.50 to carry out the emulsification under the conditions of the temperature of 80 degrees C and the stirring rotational speed of 90 rpm. Subsequently, the component (D') was blended. Thereafter, further constant-quantity dripping of the aqueous phase of the rest of the deionized water, which was heated to 80 degrees C, was carried out by spending 35 minutes while the emulsification under the conditions of the temperature of 80 degrees C and the stirring rotational speed of 90 rpm were conducted. After the emulsification was completed, the solution was cooled to 30 degrees C with a refrigerant of 5 degrees C to obtain the premixed composition.

(Example 13)

The formulation of Table 3 was converted so that the total quantity of the premix is 110 kg, and the respective components were weighed.

The components (A), (B), (C), (D) and (E) were supplied to a blending vessel (Super Mix Impeller MR205, commercially available from Satake Chemical Equipment Mfg Ltd.), and were heated to 80 degrees C while being stirred to completely dissolve the raw materials. A constant-quantity dripping of an aqueous phase of deionized water, which was heated to 80 degrees C, in this oil phase was carried out by spending 60 minutes to carry out the emulsification under the conditions of the temperature of 80 degrees C and the stirring rotational speed of 45 rpm. After the emulsification was completed, the solution was cooled to 30 degrees C with a refrigerant of 5 degrees C. Thereafter, the component (D') was added, and then the solution was stirred at a stirring rotational speed of 45 rpm for five minutes to obtain a premixed composition.

(Example 14)

A premixed composition was obtained according to Example 13, except that the stirring rotational speed was changed to 65 rpm in the emulsification and after blending the component (D').

(Example 15)

A premixed composition was obtained according to Example 13, except that the stirring rotational speed was changed to 85 rpm in the emulsification and after blending the component (D').

(Comparative Example 1)

A premixed composition was obtained according to Example 9, except that the component (D') was removed.

(Comparative Example 2)

A premixed composition was obtained according to Example 4, except that the component (D') was removed and that the formulation of the component (D) and the deionized water was adjusted as described in Table 3.

(Comparative Example 3)

A premixed composition was obtained according to Example 1, except that the component (D) was removed from the oil phase and that the formulation of Table 3 was converted so that the total quantity of the premix is 1,800 g, and the respective components were weighed.

(Comparative Example 4)

The formulation of Table 3 was converted so that the total quantity of the premix is 1,800 g, and the respective components were weighed. The components (A), (B), (C), (D) and (E) were supplied to a separable flask of 2 L, and were heated to 80 degrees C while being stirred to completely dissolve the raw materials. An aqueous phase of deionized water, which was heated to 80 degrees C, in this oil phase was carried out by spending 5 minutes until the ratio of aqueous phase (W) and oil phase (O) reaches 0.50 to carry out the emulsification under the conditions of the temperature of 80 degrees C and the stirring rotational speed of 90 rpm. Subsequently, the component (D') was blended. Thereafter, further constant-quantity dripping of the aqueous phase of the rest of the deionized water, which was heated to 80 degrees C, was carried out by spending 55 minutes while the emulsification under the conditions of the temperature of 80 degrees C and the stirring rotational speed of 90 rpm were conducted. After the emulsification was completed, the solution was cooled to 30 degrees C with a refrigerant of 5 degrees C to obtain the premixed composition.

[Table 1]

TABLE 1

| | | UNIT | EXAMPLE 1 | EXAMPLE 2 | EXAMPLE 3 | EXAMPLE 4 | EXAMPLE 5 | EXAMPLE 6 |
|---|---|---|---|---|---|---|---|---|
| FORMULATION(% BY MASS) | (A) PALMITIC ACID *1 | [wt%] | 0.47 | 0.47 | 0.47 | 0.55 | 0.47 | 0.47 |
| | (A) STEARIC ACID *2 | [wt%] | 0.52 | 0.52 | 0.52 | 0.82 | 0.52 | 0.52 |
| | (A) ISOSTEARICACID *3 | [wt%] | 0.65 | 0.65 | 0.65 | 0.48 | 0.65 | 0.65 |
| | (A) BEHENIC ACID *4 | [wt%] | 0.16 | 0.16 | 0.16 | 0.15 | 0.16 | 0.16 |
| | (B) N,N-DIMETHYLOCTADECYLOXY PROPYLAMINE *5 | [wt%] | 3.18 | 3.18 | 2.89 | 3.44 | 3.18 | 3.18 |
| | (B) N-(3(DIMETHYL-AMINO)PROPYL)DO-COSANAMIDE *6 | [wt%] | - | - | - | - | - | - |
| | (C) LACTIC ACID *7 | [wt%] | 0.17 | 0.17 | 0.17 | 0.17 | 0.17 | 0.17 |
| | (D) DIPROPYLENE GLYCOL *8 | [wt%] | 6.00 | 6.00 | 6.00 | 6.00 | 6.00 | 6.00 |
| | (E) STEARYL STEARATE *9 | [wt%] | 0.20 | - | 0.20 | - | 0.20 | 0.20 |
| | (E) HYDROGENATED JOJOBA OIL *10 | [wt%] | - | 0.20 | - | - | - | - |
| | DEIONIZED WATER | [wt%] | 73.66 | 73.66 | 73.94 | 73.39 | 73.66 | 83.66 |
| | (D') DIPROPYLENE GLYCOL *8 | [wt%] | 15.00 | 15.00 | 15.00 | 15.00 | - | 5.00 |
| | (D') PROPYLENE GLYCOL *11 | [wt%] | - | - | - | - | 15.00 | - |
| | PREMIX TOTAL | [wt%] | 100.00 | 100.00 | 100.00 | 100.00 | 100.00 | 100.00 |

(continued)

| | | UNIT | EXAMPLE 1 | EXAMPLE 2 | EXAMPLE 3 | EXAMPLE 4 | EXAMPLE 5 | EXAMPLE 6 |
|---|---|---|---|---|---|---|---|---|
| CONDITIONS FOR PREPARING PREMIX | EMULSIFICATION METHOD | [-] | PHASE INVERSION | PHASE INVERSION | PHASE INVERSION | PHASE INVERSION | PHASE INVERSION | PHASE INVERSION |
| | DEGREE OF NEUTRALIZATION: {(A)+(C)}/(B) | [MOLAR RATIO] | 1.00 | 1.00 | 1.10 | 1.00 | 1.00 | 1.00 |
| | EMULSIFICATION TEMPERATURE | [°C] | 80 | 80 | 80 | 80 | 80 | 80 |
| | ROTATIONAL SPEED | [rpm] | 90 | 90 | 90 | 90 | 90 | 90 |
| | TIME FOR DROPPING AQUEOUS PHASE | [min] | 60 | 60 | 60 | 60 | 60 | 60 |
| | W/O RATIO AT START OF ADDITION OF (D') | [WEIGHT RATIO] | 6.49 | 6.49 | 6.69 | 6.32 | 6.49 | 7.37 |
| | TEMPERATURE IN VESSEL AT START OF ADDITION OF (D') | [°C] | 30 | 30 | 30 | 30 | 30 | 30 |
| RINSE FORMULATION | DEIONIZED WATER | [g] | 301.40 | 301.40 | 301.40 | 301.40 | 301.40 | 301.40 |
| | LACTIC ACID | [g] | 2.36 | 2.36 | 2.36 | 2.36 | 2.36 | 2.36 |
| | N,N-DIMETHYLOCTADECYLOXY PROPYLAMINE | [g] | 9.29 | 9.29 | 9.29 | 9.29 | 9.29 | 9.29 |
| | STEARYLALCOHOL | [g] | 21.00 | 21.00 | 21.00 | 21.00 | 21.00 | 21.00 |
| | DIPROPYLENE GLYCOL | [g] | 5.95 | 5.95 | 5.95 | 5.95 | 5.95 | 5.95 |
| | PREMIX | [g] | 10.00 | 10.00 | 10.00 | 10.00 | 10.00 | 10.00 |
| | RINSE TOTAL | [g] | 350.00 | 350.00 | 350.00 | 350.00 | 350.00 | 350.00 |

(continued)

| | | UNIT | EXAMPLE 1 | EXAMPLE 2 | EXAMPLE 3 | EXAMPLE 4 | EXAMPLE 5 | EXAMPLE 6 |
|---|---|---|---|---|---|---|---|---|
| EVALUATION RESULTS | MICROSCOPIC OBSERVATION | [JUST AFTER PREPARATION] | ◎ | ◎ | ◎ | ◎ | ◎ | ◎ |
| | | [STORAGE AT RT] | ◎ | ◎ | ◎ | ○ | ◎ | ◎ |
| | | [STORAGE AT 50 DEGREE C] | ◎ | ○ | ○ | Δ | ◎ | ◎ |
| | | [STORAGE AT -10 DEGREE C] | O | ○ | ◎ | ○ | ◎ | ○ |
| | SMOOTHNESS AND ABSENCE OF SLIMY FEEL IN RINSING, AND, SMOOTHNESS AND ABSENCE OF PASTINESS AFTER DRYING (AVERAGE SCORE) | [-] | 4.4 | 3.9 | 4.7 | 3.7 | 4.7 | 4.4 |

◎ (DOUBLE CIRCLES): CLEAR MALTESE CROSS DERIVED FROM THE VESICLE STRUCTURE IS OBSERVED.
○ (SINGLE CIRCLE): MALTESE CROSS DERIVED FROM THE VESICLE STRUCTURE IS OBSERVED.
Δ (TRIANGLE): MALTESE CROSS DERIVED FROM THE VESICLE STRUCTURE IS SLIGHTLY OBSERVED.
× (CROSS): NONE OF MALTESE CROSS DERIVED FROM THE VESICLE STRUCTURE IS OBSERVED.

[Table 2]

TABLE 2

| | | UNIT | EXAMPLE 7 | EXAMPLE 8 | EXAMPLE 9 | EXAMPLE 10 | EXAMPLE 11 | EXAMPLE 12 |
|---|---|---|---|---|---|---|---|---|
| FORMULATION (% BY MASS) | (A) PALMITIC ACID *1 | [wt%] | 0.47 | 0.47 | - | - | 0.47 | 0.47 |
| | (A) STEARIC ACID *2 | [wt%] | 0.52 | 0.52 | 120 | 1.80 | 0.52 | 0.52 |
| | (A) ISOSTEARIC ACID *3 | [wt%] | 0.65 | 0.65 | 0.80 | - | 0.65 | 0.65 |
| | (A) BEHENIC ACID *4 | [wt%] | 0.16 | 0.16 | - | - | 0.16 | 0.16 |
| | (B) N,N-DIMETHYLOCTADECYLOXY PROPYLAMINE *5 | [wt%] | 3.18 | - | 3.44 | 3.12 | 3.18 | 3.18 |
| | (B) N-(3-(DIMETHYL-AMINO)PROPYL)DO-COSANAMIDE *6 | [wt%] | - | 3.45 | - | - | - | - |
| | (C) LACTIC ACID *7 | [wt%] | 0.17 | 0.17 | 0.17 | 0.16 | 0.17 | 0.17 |
| | (D) DIPROPYLENE GLYCOL *8 | [wt%] | 6.00 | 6.00 | 6.00 | 6.00 | 6.00 | 6.00 |
| | (E) STEARYL STEARATE *9 | [wt%] | 020 | 0.20 | - | 020 | 0.20 | 0.20 |
| | (E) HYDROGENATED JOJOBA OIL *10 | [wt%] | - | - | - | - | - | - |
| | DEIONIZED WATER | [wt%] | 78.66 | 78.38 | 73.39 | 78.72 | 73.66 | 73.66 |
| | (D') DIPROPYLENE GLYCOL *8 | [wt%] | 5.00 | 5.00 | 15.00 | 5.00 | 15.00 | 15.00 |
| | (D') PROPYLENE GLYCOL *11 | [wt%] | 5.00 | 5.00 | - | 5.00 | - | - |
| | PREMIX TOTAL | [wt%] | 100.00 | 100.00 | 100.00 | 100.00 | 100.00 | 100.00 |

(continued)

| | | UNIT | EXAMPLE 7 | EXAMPLE 8 | EXAMPLE 9 | EXAMPLE 10 | EXAMPLE 11 | EXAMPLE 12 |
|---|---|---|---|---|---|---|---|---|
| CONDITIONS PREPARING PREMIX | EMULSIFICATION METHOD | [-] | PHASE INVERSION | PHASE INVERSION | PHASE INVERSION | PHASE INVERSION | PHASE INVERSION | PHASE INVERSION |
| | DEGREE OF NEUTRALIZATION: {(A)+(C)}/(B) | [MOLAR RATIO] | 1.00 | 1.00 | 1.00 | 1.00 | 1.00 | 1.00 |
| | EMULSIFICATION TEMPERATURE | [°C] | 80 | 80 | 80 | 80 | 80 | 80 |
| | ROTATIONAL SPEED | [rpm] | 90 | 90 | 90 | 90 | 90 | 90 |
| | TIME FOR DROPPING AQUEOUS PHASE | [min] | 60 | 60 | 60 | 60 | 60 | 60 |
| | W/O RATIO AT START OF ADDITION OF (D') | [WEIGHT RATIO] | 6.93 | 6.75 | 6.32 | 6.98 | 6.49 | 2.50 |
| | TEMPERATURE IN VESSEL AT START OF ADDITION OF (D') | [°C] | 30 | 30 | 30 | 30 | 70 | 80 |
| RINSE FORMULATION | DEIONIZED WATER | [g] | 301.40 | 301.40 | 301.40 | 301.40 | 301.40 | 301.40 |
| | LACTIC ACID | [g] | 2.36 | 2.36 | 2.36 | 2.36 | 2.36 | 2.36 |
| | N,N-DIMETHYLOCTADECYLOXY PROPYLAMINE | [g] | 929 | 929 | 929 | 9.29 | 9.29 | 9.29 |
| | STEARYLALCOHOL | [g] | 21.00 | 21.00 | 21.00 | 21.00 | 21.00 | 21.00 |
| | DIPROPYLENE GLYCOL | [g] | 5.95 | 5.95 | 5.95 | 5.95 | 5.95 | 5.95 |
| | PREMIX | [g] | 10.00 | 10.00 | 10.00 | 10.00 | 10.00 | 10.00 |
| | RINSE TOTAL | [g] | 350.00 | 350.00 | 350.00 | 350.00 | 350.00 | 350.00 |

(continued)

| | | UNIT | EXAMPLE 7 | EXAMPLE 8 | EXAMPLE 9 | EXAMPLE 10 | EXAMPLE 11 | EXAMPLE 12 |
|---|---|---|---|---|---|---|---|---|
| EVALUATION RESULTS | MICROSCOPIC OBSERVATION | [JUST AFTER PREPARATION] | ◎ | ◎ | ◎ | ◎ | ◎ | ◎ |
| | | [STORAGE AT RT] | ◎ | ◎ | ○ | ◎ | ◎ | ◎ |
| | | [STORAGE AT 50 DEGREE C] | ◎ | ○ | Δ | ○ | ◎ | ◎ |
| | | [STORAGE AT -10 DEGREE C] | ◎ | ○ | ○ | ○ | ○ | ○ |
| | SMOOTHNESS AND ABSENCE OF SLIMY FEEL IN RINSING, AND, SMOOTHNESS AND ABSENCE OF PASTINESS AFTER DRYING (AVERAGE SCORE) | [-] | 4.9 | 4.4 | 3.9 | 4.1 | 4.2 | 4.3 |

◎ (DOUBLE CIRCLES): CLEAR MALTESE CROSS DERIVED FROM THE VESICLE STRUCTURE IS OBSERVED.
○ (SINGLE CIRCLE): MALTESE CROSS DERIVED FROM THE VESICLE STRUCTURE IS OBSERVED.
Δ (TRIANGLE): MALTESE CROSS DERIVED FROM THE VESICLE STRUCTURE IS SLIGHTLY OBSERVED.
× (CROSS): NONE OF MALTESE CROSS DERIVED FROM THE VESICLE STRUCTURE IS OBSERVED.

[Table 3]

| TABLE 3 | | | INFLUENCE OF SCALE | | |
|---|---|---|---|---|---|
| | | UNIT | EXAMPLE 13 | EXAMPLE 14 | EXAMPLE 15 |
| FORMULATION (% BY MASS) | (A) PALMITIC ACID *1 | [wt%] | 0.47 | 0.47 | 0.47 |
| | (A) STEARICACID *2 | [wt%] | 0.52 | 0.52 | 0.52 |
| | (A) ISOSTEARICACID *3 | [wt%] | 065 | 065 | 065 |
| | (A) BEHENICACID*4 | [wt%] | 0.16 | 0.16 | 0.16 |
| | (B) N,N-DIMETHYLOCTADECYLOXY PROPYLAMINE *5 | [wt%] | 3.18 | 3.18 | 3.18 |
| | (B) N-(3-(DIMETHYLAMINO)PROPYL)DOCOSANAMIDE *6 | [wt%] | - | - | - |
| | (C) LACTIC ACID *7 | [wt%] | 0.17 | 0.17 | 0.17 |
| | (D) DIPROPYLENE GLYCOL *8 | [wt%] | 8.00 | 8.00 | 8.00 |
| | (E) STEARYL STEARATE *9 | [wt%] | 0.20 | 0.20 | 0.20 |
| | (E) EXTREMELY HYDROGENATED JOJOBA OIL *10 | [wt%] | - | - | - |
| | DEIONIZED WATER | [wt%] | 78.66 | 78.66 | 78.66 |
| | (D') DIPROPYLENE GLYCOL *8 | [wt%] | 3.00 | 3.00 | 3.00 |
| | (D') PROPYLENE GLYCOL *11 | [wt%] | 5.00 | 5.00 | 5.00 |
| | PREMIX TOTAL | [wt%] | 100.00 | 100.00 | 100.00 |

(continued)

| TABLE 3 | | | INFLUENCE OF SCALE | | |
|---|---|---|---|---|---|
| | | UNIT | EXAMPLE 13 | EXAMPLE 14 | EXAMPLE 15 |
| CONDITIONS FOR PREPARING PREMIX | TOTAL LOADING QUANTITY IN BLENDING VESSEL | [kg] | 110.00 | 110.00 | 110.00 |
| | EMULSIFICATION METHOD | [-] | PHASE INVERSION | PHASE INVERSION | PHASE INVERSION |
| | DEGREE OF NEUTRALIZATION: {(A)+(C)}/(B) | [MOLAR RATIO] | 1.00 | 1.00 | 1.00 |
| | EMULSIFICATION TEMPERATURE | [°C] | 80 | 80 | 80 |
| | TYPE OF IMPELLER | [-] | SUPER MIX IMPELLER | SUPER MIX IMPELLER | SUPER MIX IMPELLER |
| | ROTATIONAL SPEED | [rpm] | 45 | 65 | 85 |
| | TIME FOR DROPPING AQUEOUS PHASE | [min] | 60 | 60 | 60 |
| | POWER CONSUMPSION: PV | [$kW/m^3$] | 0.19 | 0.87 | 1.73 |
| | W/O RATIO AT START OF ADDITION OF (D') | [WEIGHT RATIO] | 5.89 | 5.89 | 5.89 |
| | TEMPERATURE IN VESSEL AT START OF ADDITION OF (D') | [°C] | 30 | 30 | 30 |
| RINSE FORMULATION | DEIONIZED WATER | [g] | 301.40 | 301.40 | 301.40 |
| | LACTICACID | [g] | 2.36 | 2.36 | 2.38 |
| | N,N-DIMETHYLOCTADECYLOXY PROPYLAMINE | [g] | 9.29 | 9.29 | 9.29 |
| | STEARYLALCOHOL | [g] | 21.00 | 21.00 | 21.00 |
| | DIPROPYLENE GLYCOL | [g] | 5.95 | 5.95 | 5.95 |
| | PREMIX | [g] | 10.00 | 10.00 | 10.00 |
| | RINSETOTAL | [g] | 350.00 | 350.00 | 350.00 |

(continued)

| TABLE 3 | | | INFLUENCE OF SCALE | | |
|---|---|---|---|---|---|
| | | UNIT | EXAMPLE 13 | EXAMPLE 14 | EXAMPLE 15 |
| EVALUATION RESULTS | MICROSCOPIC OBSERVATION | [JUST AFTER PREPARATION] | ◎ | ◎ | ◎ |
| | | [STORAGE AT RT] | ◎ | ◎ | ◎ |
| | | [STORAGE AT 50 DEGREE C] | ◎ | ◎ | ◎ |
| | | [STORAGE AT -10 DEGREE C] | ◎ | ◎ | ◎ |
| | SMOOTHNESS AND ABSENCE OF SLIMY FEEL IN RINSING, AND, SMOOTHNESS AND ABSENCE OF PASTINESS AFTER DRYING (AVERAGE SCORE) | [-] | 49 | 48 | 4.7 |

◎ (DOUBLE CIRCLES): CLEAR MALTESE CROSS DERIVED FROM THE VESICLE STRUCTURE IS OBSERVED.
○ (SINGLE CIRCLE): MALTESE CROSS DERIVED FROM THE VESICLE STRUCTURE IS OBSERVED.
Δ (TRIANGLE): MALTESE CROSS DERIVED FROM THE VESICLE STRUCTURE IS SLIGHTLY OBSERVED.
× (CROSS): NONE OF MALTESE CROSS DERIVED FROM THE VESICLE STRUCTURE IS OBSERVED.

[Table 4]

TABLE 4

| | | UNIT | COMPARATIVE EXAMPLE 1 | COMPARATIVE EXAMPLE 2 | COMPARATIVE EXAMPLE 3 | COMPARATIVE EXAMPLE 4 |
|---|---|---|---|---|---|---|
| FORMULATION (% BY MASS) | (A) PALMITIC ACID *1 | [wt%] | - | 0.55 | 0.47 | 0.47 |
| | (A) STEARIC ACID *2 | [wt%] | 1.20 | 0.82 | 0.52 | 0.52 |
| | (A) ISOSTEARIC ACID *3 | [wt%] | 0.80 | 0.48 | 0.65 | 0.65 |
| | (A) BEHENIC ACID *4 | [wt%] | - | 0.15 | 0.16 | 0.16 |
| | (B) N,N-DIMETHYLOCTADECYLOXY PROPYLAMINE *5 | [wt%] | 3.44 | 3.44 | 3.18 | 3.18 |
| | (B) N-(3-(DIMETHYLAMINO)PROPYL)DOCOSAN AMIDE *6 | [wt%] | - | - | - | - |
| | (C) LACTIC ACID *7 | [wt%] | 0.17 | 0.17 | 0.17 | 0.17 |
| | (D) DIPROPYLENE GLYCOL *8 | [wt%] | 6.00 | 11.00 | - | 6.00 |
| | (E) STEARYL STEARATE *9 | [wt%] | - | - | 0.20 | 0.20 |
| | (E) HYDROGENATED JOJOBA OIL *10 | [wt%] | - | - | - | - |
| | DEIONIZED WATER | [wt%] | 88.39 | 83.39 | 79.66 | 78.66 |
| | (D') DIPROPYLENE GLYCOL *8 | [wt%] | - | - | 15.00 | 5.00 |
| | (D') PROPYLENE GLYCOL *11 | [wt%] | - | - | - | 5.00 |
| | PREMIX TOTAL | [wt%] | 100.00 | 100.00 | 100.00 | 100.00 |
| CONDITIONS FORPREPARING PREMIX | EMULSIFICATION METHOD | [-] | PHASE INVERSION | PHASE INVERSION | PHASE INVERSION | PHASE INVERSION |
| | DEGREE OF NEUTRALIZATION: {(A)+{C)}/(B) | [MOLAR RATIO] | 1.00 | 1.00 | 1.00 | 1.00 |
| | EMULSIFICATION TEMPERATURE | [°C] | 80 | 80 | 80 | 80 |
| | ROTATIONAL SPEED | [rpm] | 90 | 90 | 90 | 90 |
| | TIME FOR DROPPING AQUEOUS PHASE | [min] | 60 | 60 | 60 | 60 |
| | W/O RATIO AT START OF ADDITION OF (D') | [WEIGHT RATIO] | - | - | 14.90 | 0.50 |
| | TEMPERATURE IN VESSELAT START OF ADDITION OF (D') | [°C] | - | - | 30 | 80 |

(continued)

| | | UNIT | COMPARATIVE EXAMPLE 1 | COMPARATIVE EXAMPLE 2 | COMPARATIVE EXAMPLE 3 | COMPARATIVE EXAMPLE 4 |
|---|---|---|---|---|---|---|
| RINSE FORMULATION | DEIONIZED WATER | [g] | 301.40 | 301.40 | 301.40 | 301.40 |
| | LACTIC ACID | [g] | 2.36 | 2.36 | 2.36 | 2.36 |
| | N,N-DIMETHYLOCTADECYLOXY PROPYLAMINE | [g] | 929 | 9.29 | 9.29 | 9.29 |
| | STEARYLALCOHOL | [g] | 21.00 | 21.00 | 21.00 | 21.00 |
| | DIPROPYLENEGLYCOL | [g] | 5.95 | 5.95 | 5.95 | 5.95 |
| | PREMIX | [g] | 10.00 | 10.00 | 10.00 | 10.00 |
| | RINSE TOTAL | [g] | 350.00 | 350.00 | 350.00 | 350.00 |
| EVALUATION RESULTS | MICROSCOPIC OBSERVATION | [JUSTAFTER PREPARATION] | ◎ | ○ | ○ | × |
| | | [STORAGE AT RT] | ○ | ○ | ○ | × |
| | | [STORAGEAT 50 DEGREE C] | × | Δ | × | × |
| | | [STORAGEAT -10 DEGREE C] | × | x | × | × |
| | SMOOTHNESS AND ABSENCE OF SLIMY FEEL IN RINSING, AND, SMOOTHNESS AND ABSENCE OF PASTINESS AFTER DRYING (AVERAGE SCORE) | [-] | 1.9 | 2.2 | 2.1 | 1.5 |

◎ (DOUBLE CIRCLES): CLEAR MALTESE CROSS DERIVED FROM THE VESICLE STRUCTURE IS OBSERVED.
○ (SINGLE CIRCLE): MALTESE CROSS DERIVED FROM THE VESICLE STRUCTURE IS OBSERVED.
Δ (TRIANGLE): MALTESE CROSS DERIVED FROM THE VESICLE STRUCTURE IS SLIGHTLY OBSERVED.
× (CROSS): NONE OF MALTESE CROSS DERIVED FROM THE VESICLE STRUCTURE IS OBSERVED.

The following raw materials were employed in formulae of the above-described Table 1 to Table 4.

*1 palmitic acid: LUNAC P-95 (molecular weight 256.4, commercially available from Kao Corporation)
* 2 stearic acid: LUNAC S-90V (molecular weight 284.48, commercially available from Kao Corporation)
* 3 isostearic acid: ISOSTEARIC ACID EX (molecular weight 284.48, commercially available from a KOKYU ALCOHOL KOGYO CO., LTD.)
* 4 behenic acid: LUNAC BA (molecular weight 340.58, commercially available from Kao Corporation)
* 5 N,N-dimethyloctadecyloxy propylamine: FARMIN DM E-80 (degree of purity 90%, molecular weight 355.63, commercially available from Kao Corporation)
* 6 N-(3-(dimethylamino)propyl)docosane amide: AMIDET APA-22 (molecular weight 424.8, commercially available from Kao Corporation)
* 7 lactic acid: MUSASHINO LACTIC ACID 90 (lactic acid, degree of purity 90%, molecular weight 90.08: melting point 18 degrees C, commercially available from Musashino Chemical Laboratory Ltd.)
* 8 dipropylene glycol: DPG-RF (commercially available from ADEKA)
* 9 stearyl stearate: EXCEPARL SS (commercially available from Kao Corporation)
* 10 hydrogenated jojoba oil: extremely hydrogenated jojoba oil (commercially available from Koei Kogyo Co., Ltd.)
* 11 propylene glycol: propylene glycol for cosmetics (commercially available from ADEKA)

(Evaluations of Premixes)

The stabilities of the vesicles in the premixed compositions obtained in Examples 1 to 15 and Comparative Examples 1 to 4 were respectively evaluated at the timings of: just after the preparation, after the storage for one month at a room temperature, after the storage at higher temperature (50 degrees C) and after the storage at lower temperature (-10 degrees C).

(1) Evaluations of the stability in the storage at higher temperature

The premixed compositions obtained in Examples 1 to 15 and Comparative Examples 1 to 4 were stored at the condition at 50 degrees C for one month. The storage stability at higher temperature was evaluated by identifying an existence or nonexistence of the vesicle in the sample after storage by employing a polarization microscopy.

◎ (double circles): clear Maltese Cross derived from the vesicle structure was observed.
○ (single circle): Maltese Cross derived from the vesicle structure was observed.
Δ (triangle): Maltese Cross derived from the vesicle structure was slightly observed.
× (cross): none of Maltese Cross derived from the vesicle structure was observed.

(2) Evaluation of storage stability at lower temperature

The premixed compositions obtained in Examples 1 to 15 and Comparative Examples 1 to 4 were stored at the condition at -10 degrees C for one month. The storage stability at higher temperature was evaluated by identifying an existence or nonexistence of the vesicle in the sample after storage by employing a polarization microscopy.

◎ (double circles): clear Maltese Cross derived from the vesicle structure was observed.
○ (single circle): Maltese Cross derived from the vesicle structure was observed.
Δ (triangle): Maltese Cross derived from the vesicle structure was slightly observed.
× (cross): none of Maltese Cross derived from the vesicle structure was observed.

In addition to above, the existences or non-existences of the vesicles were evaluated for the samples at the timings of: just after the preparation, after the storage for one month at a room temperature, according to the above-described criteria for the evaluations.

(Preparation of Rinse)

Rinses were prepared by employing the premixed compositions obtained in Examples 1 to 15 and Comparative Examples 1 to 4. 301.40 g of an aqueous phase of deionized water and 2.36 g of MUSASHINO LACTIC ACID 90 (lactic acid, degree of purity 90%, molecular weight 90.08: melting point 18 degrees C, commercially available from Musashino Chemical Laboratory Ltd.) were supplied in a 500 mL beaker, and the mixture was heated to 55 degrees C while being stirred with a propeller. Thereafter, an oil phase composed of 9.29 g of FARMIN DME-80 (N,N-dimethyloctadecyloxy

propylamine, degree of purity 90%, molecular weight 355.63, commercially available from Kao Corporation), 21.00 g of KALCOL 8098 (stearyl alcohol, commercially available from Kao Corporation) and 5.95 g of DPG-RF (dipropylene glycol, commercially available from ADEKA) was uniformly dissolved at 80 degrees C, and then was added in the aqueous phase, and was stirred at 300 rpm for 10 minutes to carry out emulsification. It was radiationally-cooled to a temperature of equal to or lower than 35 degrees C to prepare a base rinse serving as a hair cosmetic composition base, and 10.00 g of the premixed compositions obtained in Examples 1 to 15 and Comparative Examples 1 to 4 were added to produce rinses. In addition to above, the premixed composition to be added was that stored for one month under the condition of -10 degrees C.

(Method for Evaluating Rinses)

20 g of hair strands (about 15.20 cm) provided by Japanese women and treated with cosmetic treatments such as cold-waving, hair-bleaching and the like, were tied together, and was cleaned with the shampoo. This hair was uniformly applied with 2 g of the rinse prepared in each of Examples 1 to 15 and Comparative Examples 1 to 4, and was rinsed off with running water for 30 seconds, and then towel drying was conducted, and further drying was conducted with a hair dryer, and general sensory evaluations of the resultant hair were conducted in relation to: "smoothness and absence of slimy feel in rinsing, and, smoothness and absence of pastiness after drying".

The evaluations were made by 10 specialty panelists with 5 scale-evaluations, and were averaged. Products having averaged evaluations of equal to or higher than 3 were classified as acceptable products.

(Criteria for Evaluations)

"smoothness and absence of slimy feel in rinsing, and, smoothness and absence of pastiness after drying"

5: "superior in the balance among the smoothness and the absence of the slimy feel in rinsing, and, the smoothness and the absence of the pastiness after drying"
4: "better in the balance among the smoothness and the absence of the slimy feel in rinsing, and, the smoothness and the absence of the pastiness after drying"
3: "slightly good in the balance among the smoothness and the absence of the slimy feel in rinsing, and, the smoothness and the absence of the pastiness after drying"
2: "slightly inferior in the balance among smoothness and the absence of the slimy feel in rinsing, and, the smoothness and the absence of the pastiness after drying"
1: "inferior in the balance among smoothness and the absence of the slimy feel in rinsing, and, the smoothness and the absence of the pastiness after drying"

In the above-described Examples, a polyol was blended in the vesicle composition, a portion of which was previously blended in the oil phase as the component (D) and the rest of which was added at a specific stage as the component (D'), so that enhanced storage stability at lower temperature of around -10 degrees C of the obtained vesicle composition was achieved.

Also, the vesicle compositions obtained in the above-described Examples exhibited enhanced balance between the storage stability at lower temperature and the storage stability at higher temperature.

Also, since the rinses obtained in the above-described Examples contain the vesicle compositions obtained by the specific method, the smoothness of the hair was achieved in the rinsing or in the drying, even if the hair was the damaged hair. Also, the slimy feel in the rinsing or the pastiness after the drying were effectively suppressed by the uses of the rinses obtained in the above-described Examples.

In relation to Example 4 and Comparative Example 1, viscosities were measured for the sample just after the preparation, the sample stored for one month at 50 degrees C, and the sample stored for one month at -10 degrees. A Brookfield viscometer manufactured by TOKI SANGYO CO., LTD (TV-10) was employed to measure the viscosity under the conditions of rotor Nos. 2 to 4 and rotational speed of 30 rpm. In addition to above, the described viscosities are the results of the measurements, in which the stored samples were allowed to stand in a warm bath of 30 degrees C for 3 hours and then the measurements were made. The measurement results are shown in Table 5.

According to Table 5, the viscosity change was smaller in Example as compared with Comparative Examples, which indicates that the storage stability was improved.

[Table 5]

TABLE 5

| | | EXAMPLE 4 | COMPARATIVE EXAMPLE 1 |
|---|---|---|---|
| JUST AFTER PREPARATION | **[mPa·s]** | **134**※1 | **12**※1 |
| ONE MONTH AT 50 DEGREES C | **[mPa·s]** | **355**※1 | **681**※1 |
| ONE MONTH AT -10 DEGREES C | **[mPa·s]** | **837**※1 | **5390**※2 |
| ※1 MEASURED VALUE WITH ROTOR NO. 2<br>※2 MEASURED VALUE WITH ROTOR NO. 4 | | | |

(Reference Examples 1 to 20)

The following methods were conducted to prepare premixed compositions for the respective Examples. Also, the obtained premixed compositions were employed to conduct preparations and evaluations of rinses. Formulations of the premixes and the rinses, production conditions for the premixes, and the evaluation results of the rinse are shown in Table 6 to Table 9.

(Reference Example 1)

3.85 kg of 18-MEA (mixture of fatty acid and branched fatty acid containing 18-methyl eicosanic acid, average molecular weight: 364.3, melting point: 35 to 55 degrees C, commercially available from Croda Japan KK), 0.26 kg of MUSASHINO LACTIC ACID 90 (lactic acid, degree of purity 90%, molecular weight 90.08: melting point 18 degrees C, commercially available from Musashino Chemical Laboratory Ltd.), 5.22 kg of FARMIN DM E-80 (N,N-dimethyloctadecyloxy propylamine, degree of purity 90%, molecular weight 355.63, commercially available from Kao Corporation), and 11.55 kg of DPG-RF (dipropylene glycol, melting point -40 degrees C, commercially available from ADEKA) were charged to a blending vessel (T.K. AGI HOMO MIXER, commercially available from PRIMIX Corporation), and then were heated while stirring with puddle vanes until the temperature in the vessel was reached to 80 degrees C to completely dissolve the raw materials. A constant-quantity dripping of 89.12 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition.

The maximum value of the power consumption in the emulsification was directly measured by employing a clamp-type dynamometer (Clamp-on Power High Tester 3169, commercially available from HIOKI E.E. CORPORATION), and the result was 2.02 $kW/m^3$.

The gel transition temperature of the vesicle composition prepared under the above-described conditions was measured with differential scanning calorimetry (DSC), and the result was 52.7 degrees C.

In addition to above, the measurement of the gel transition temperature was conducted with "μDSC7evo" commercially available from SETARAM Instrumentation, and the measurement conditions were: from 5 degrees C to 90 degrees C and the rate of the temperature elevation was 0.5 degrees C/minute. The production conditions for the premixes and the weight fractions of the respective components were shown in Table 6 to Table 9.

(Reference Example 2)

2.20 kg of LUNAC S-90V (stearic acid, average molecular weight 284.8, commercially available from Kao Corporation), 3.82 kg of FARMIN DM E-80 (N,N-dimethyloctadecyloxy propylamine, degree of purity 90%, molecular weight 355.63, commercially available from Kao Corporation), 0.19 kg of MUSASHINO LACTIC ACID 90 (lactic acid, degree of purity 90%, molecular weight 90.08: melting point 18 degrees C, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and then were heated to 80 degrees C while stirring with puddle vanes to completely dissolve the raw materials. A constant-quantity dripping of 97.19 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 1.87 kW/$m^3$.

(Reference Example 3)

2.20 kg of LUNAC MY-98 (myristic acid, average molecular weight: 228.4 commercially available from Kao Corporation), 4.76 kg of FARMIN DM E-80 (N,N-dimethyloctadecyloxy propylamine, degree of purity 90%, molecular weight 355.63, commercially available from Kao Corporation), 0.24 kg of MUSASHINO LACTIC ACID 90 (lactic acid, degree of purity 90%, molecular weight 90.08: melting point 18 degrees C, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and then were heated to 80 degrees C while stirring with puddle vanes to completely dissolve the raw materials. A constant-quantity dripping of 96.20 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 1.93 kW/$m^3$.

(Reference Example 4)

2.20 kg of LUNAC BA (behenic acid, average molecular weight: 340.6, commercially available from Kao Corporation), 3.19 kg of FARMIN DM E-80 (N,N-dimethyloctadecyloxy propylamine, degree of purity 90%, molecular weight 355.63, commercially available from Kao Corporation), 0.16 kg of lactic acid (MUSASHINO LACTIC ACID 90, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and then were heated to 80 degrees C while stirring with puddle vanes to completely dissolve the raw materials. A constant-quantity dripping of 97.85 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 1.95 kW/$m^3$.

(Reference Example 5)

1.32 kg of LUNAC S-90V (stearic acid, average molecular weight 284.8, commercially available from Kao Corporation), 0.88 kg of ISOSTEARIC ACID EX (isostearic acid, average molecular weight: 291.2, commercially available from a KOKYU ALCOHOL KOGYO CO., LTD.), 3.78 kg of FARMIN DM E-80 (N,N-dimethyloctadecyloxy propylamine, degree of purity 90%, molecular weight 355.63, commercially available from Kao Corporation), 0.19 kg of MUSASHINO LACTIC ACID 90 (lactic acid, degree of purity 90%, molecular weight 90.08: melting point 18 degrees C, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 97.23 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 2.12 kW/$m^3$.

(Reference Example 6)

1.32 kg of LUNAC MY-98 (myristic acid, average molecular weight: 228.4 commercially available from Kao Corporation), 0.88 kg of ISOSTEARIC ACID EX (isostearic acid, average molecular weight: 291.2, commercially available from a KOKYU ALCOHOL KOGYO CO., LTD.), 4.35 kg of FARMIN DM E-80 (N,N-dimethyloctadecyloxy propylamine, degree of purity 90%, molecular weight 355.63, commercially available from Kao Corporation), 0.22 kg of MUSASHINO LACTIC ACID 90 (lactic acid, degree of purity 90%, molecular weight 90.08: melting point 18 degrees C, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 96.63 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 2.05 $kW/m^3$.

(Reference Example 7)

1.32 kg of LUNAC BA (behenic acid, average molecular weight: 340.6, commercially available from Kao Corporation), 0.88 kg of ISOSTEARIC ACID EX (isostearic acid, average molecular weight: 291.2, commercially available from a KOKYU ALCOHOL KOGYO CO., LTD.), 3.41 kg of FARMIN DM E-80 (N,N-dimethyloctadecyloxy propylamine, degree of purity 90%, molecular weight 355.63, commercially available from Kao Corporation), 0.17 kg of MUSASHINO LACTIC ACID 90 (lactic acid, degree of purity 90%, molecular weight 90.08: melting point 18 degrees C, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 97.62 kg of an aqueous phase of deionized water heated to 80 degrees C was carried out to this oil phase by spending 75 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 2.23 $kW/m^3$.

(Reference Example 8)

1.76 kg of LUNAC S-90V (stearic acid, average molecular weight 284.8, commercially available from Kao Corporation), 0.44 kg of ISOSTEARIC ACID EX (isostearic acid, average molecular weight: 291.2, commercially available from a KOKYU ALCOHOL KOGYO CO., LTD.), 3.80 kg of FARMIN DM E-80 (N,N-dimethyloctadecyloxy propylamine, degree of purity 90%, molecular weight 355.63, commercially available from Kao Corporation), 0.19 kg of MUSASHINO LACTIC ACID 90 (lactic acid, degree of purity 90%, molecular weight 90.08: melting point 18 degrees C, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 97.21 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 30 rpm. After that, the solution was cooled to equal to or lower than 30 degrees C with a refrigerant of 5 degrees C to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 1.12 $kW/m^3$.

(Reference Example 9)

1.98 kg of LUNAC S-90V (stearic acid, average molecular weight 284.8, commercially available from Kao Corporation), 0.22 kg of ISOSTEARIC ACID EX (isostearic acid, average molecular weight: 291.2, commercially available from a KOKYU ALCOHOL KOGYO CO., LTD.), 3.81 kg of FARMIN DM E-80 (N,N-dimethyloctadecyloxy propylamine, degree of purity 90%, molecular weight 355.63, commercially available from Kao Corporation), 0.19 kg of MUSASHINO LACTIC ACID 90 (lactic acid, degree of purity 90%, molecular weight 90.08: melting point 18 degrees C, commercially available from Musashino Chemical Laboratory Ltd.) and 6.60 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 97.20 kg of an aqueous phase of deionized water heated to 80 degrees C was carried out to this oil phase by spending 80 minutes, so that the total loading quantity to the blending vessel is 110 kg, to carry out the emulsification under the conditions of the temperature of 80 degrees C and the stirring rotational speed of 30 rpm. After that, the solution was cooled to equal to or lower than 30 degrees C with a refrigerant of 5 degrees C to obtain the premixed composition. The temperature after the cooling was equal to or higher than the gel transition temperature of the obtained vesicle composition.

In addition to above, the maximum value of the power consumption in the emulsification was directly measured by employing a clamp-type dynamometer (Clamp-on Power High Tester 3169, commercially available from HIOKI E.E. CORPORATION), and the result was 1.20 $kW/m^3$.

(Reference Example 10)

3.85 kg of 18-MEA (mixture of fatty acid and branched fatty acid containing 18-methyl eicosanic acid, average molecular weight: 364.3, melting point: 35 to 55 degrees C, commercially available from Croda Japan KK), 5.66 kg of AMIDET APA-22 (N-[3-(dimethylamino)propyl)docosane amide, degree of purity 99%, molecular weight 424.8, melting point 76 degrees C and commercially available from Kao Corporation), 0.26 kg of MUSASHINO LACTIC ACID 90 (lactic acid, degree of purity 90%, molecular weight 90.08: melting point 18 degrees C, commercially available from Musashino Chemical Laboratory Ltd.) and 11.55 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 88.67 kg of an aqueous phase of deionized water heated to 80 degrees C was carried out to this oil phase by spending 75 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition. The temperature after the cooling was equal to or higher than the gel transition temperature of the obtained vesicle composition.

In addition to above, the maximum value of the power consumption in the emulsification was directly measured by employing a clamp-type dynamometer (Clamp-on Power High Tester 3169, commercially available from HIOKI E.E. CORPORATION), and the result was 1.88 $kW/m^3$.

(Reference Example 11)

3.85 kg of 18-MEA (mixture of fatty acid and branched fatty acid containing 18-methyl eicosanic acid, average molecular weight: 364.3, melting point: 35 to 55 degrees C, commercially available from Croda Japan KK), 4.01 kg of FARMIN DM8098 (N,N-dimethyl-n-octadecylamine, degree of purity 98%, molecular weight 297.56, melting point 23 degrees C and commercially available from Kao Corporation), 0.26 kg of MUSASHINO LACTIC ACID 90 (lactic acid, degree of purity 90%, molecular weight 90.08: melting point 18 degrees C, commercially available from Musashino Chemical Laboratory Ltd.) and 11.55 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 90.33 kg of an aqueous phase of deionized water heated to 80 degrees C was carried out to this oil phase by spending 78 minutes, so that the total loading quantity to the blending vessel is 110 kg, to carry out the emulsification under the conditions of the temperature of 80 degrees C and the stirring rotational speed of 35 rpm. After that, the solution was cooled to equal to or lower than 30 degrees C with a refrigerant of 5 degrees C to obtain the premixed composition. The temperature after the cooling was equal to or higher than the gel transition temperature of the obtained vesicle composition.

In addition to above, the maximum value of the power consumption in the emulsification was directly measured by employing a clamp-type dynamometer (Clamp-on Power High Tester 3169, commercially available from HIOKI E.E.

CORPORATION), and the result was 1.92 kW/m$^3$.

(Reference Example 12)

The emulsification was carried out according to Reference Example 1 except that the rotational speed of the puddle vanes described in Reference Example 1 was changed to 45 rpm to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 3.31 kW/m$^3$.

(Reference Example 13)

The emulsification was carried out according to Reference Example 1 except that the rotational speed of the puddle vanes described in Reference Example 1 was changed to 60 rpm to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 4.29 kW/m$^3$.

(Reference Example 14)

The emulsification was carried out according to Reference Example 1 except that the rotational speed of the puddle vanes described in Reference Example 1 was changed to 25 rpm to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 0.45 kW/m$^3$.

(Reference Example 15)

The emulsification was carried out according to Reference Example 1 except that the time for dripping the aqueous phase described in Reference Example 1 was changed to 45 minutes to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 1.46 kW/m$^3$.

(Reference Example 16)

24.50 kg of 18-MEA (mixture of fatty acid and branched fatty acid containing 18-methyl eicosanic acid, average molecular weight: 364.3, melting point: 35 to 55 degrees C, commercially available from Croda Japan KK), 1.68 kg of MUSASHINO LACTIC ACID 90 (lactic acid, degree of purity 90%, molecular weight 90.08: melting point 18 degrees C, commercially available from Musashino Chemical Laboratory Ltd.), 33.18 kg of FARMIN DM E-80 (N,N-dimethyloctadecyloxy propylamine, degree of purity 90%, molecular weight 355.63, commercially available from Kao Corporation), and 73.50 kg of DPG-RF (dipropylene glycol, melting point -40 degrees C, commercially available from ADEKA) were supplied to the blending vessel (T.K. AGI HOMO MIXER, commercially available from PRIMIX Corporation), and were heated to 80 degrees C while stirring with puddle vanes to completely dissolve the raw materials. A constant-quantity dripping of 567.14 kg of an aqueous phase of deionized water heated to 80 degrees C was carried out to this oil phase by spending 77 minutes, so that the total loading quantity to the blending vessel is 700 kg, to carry out the emulsification under the conditions of a temperature of 80 degrees C and a stirring rotational speed of 40 rpm. After that, the solution was cooled to equal to or lower than 30 degrees C with a refrigerant of 5 degrees C to obtain the premixed composition.
In addition to above, the maximum value of the power consumption in the emulsification was directly measured by employing a clamp-type dynamometer (Clamp-on Power High Tester 3169, commercially available from HIOKI E.E. CORPORATION), and the result was 1.37 kW/m$^3$.

(Reference Example 17)

3.85 kg of 18-MEA (mixture of fatty acid and branched fatty acid containing 18-methyl eicosanic acid, average molecular weight: 364.3, melting point: 35 to 55 degrees C, commercially available from Croda Japan KK), 0.26 kg of MUSASHINO LACTIC ACID 90 (lactic acid, degree of purity 90%, molecular weight 90.08: melting point 18 degrees C, commercially available from Musashino Chemical Laboratory Ltd.), 5.22 kg of FARMIN DM E-80 (N,N-dimethyloctadecyloxy propylamine, degree of purity 90%, molecular weight 355.63, commercially available from Kao Corporation) and 11.55 kg of DPG-RF (dipropylene glycol, melting point -40 degrees C, commercially available from ADEKA) were supplied to a blending vessel (SUPER MIX MR205, commercially available from Satake Chemical Equipment Mfg Ltd.), and were heated to 80 degrees C while stirring with puddle vanes to completely dissolve the raw materials. A constant-quantity dripping of 89.12 kg of an aqueous phase of deionized water heated to 80 degrees C was carried out to this oil phase by spending 30 minutes, so that the total loading quantity to the blending vessel is 110 kg, to carry out the emulsification under the conditions of a temperature of 80 degrees C and a stirring rotational speed of 90 rpm. After that, the solution was cooled to equal to or lower than 30 degrees C with a refrigerant of 5 degrees C to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 1.53 kW/$m^3$.

(Reference Example 18)

The emulsification was carried out according to Reference Example 1 except that the rotational speed of the puddle vanes described in Reference Example 1 was changed to 10 rpm and the constant-quantity dripping of the heated deionized water was carried out by spending 71 minutes, to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 0.08 kW/$m^3$.

(Reference Example 19)

The emulsification was carried out according to Reference Example 1 except that the rotational speed of the puddle vanes described in Reference Example 1 was changed to 80 rpm and the constant-quantity dripping of the heated deionized water was carried out by spending 15 minutes, to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 11.0 kW/$m^3$.

(Reference Example 20)

89.12 kg of an aqueous phase of deionized water was supplied to the blending vessel (T.K. AGI HOMO MIXER, commercially available from PRIMIX Corporation) so that the total loading quantity to the blending vessel is 110 kg, and was heated to 80 degrees C while stirring with puddle vanes to completely dissolve the raw materials, and after that, an oil phase, which is obtained by completely dissolving the raw materials at 80 degrees C, which contains 3.85 kg of 18-MEA (mixture of fatty acid and branched fatty acid containing 18-methyl eicosanic acid, average molecular weight: 364.3, melting point: 35 to 55 degrees C, commercially available from Croda Japan KK), 0.26 kg of MUSASHINO LACTIC ACID 90 (lactic acid, degree of purity 90%, molecular weight 90.08: melting point 18 degrees C, commercially available from Musashino Chemical Laboratory Ltd.), 5.22 kg of FARMIN DM E-80 (N,N-dimethyloctadecyloxy propylamine, degree of purity 90%, molecular weight 355.63, commercially available from Kao Corporation) and 11.55 kg of DPG-RF (dipropylene glycol, melting point -40 degrees C, commercially available from ADEKA), was employed to carry out a constant-quantity dripping of the oil phase to an aqueous phase continued in the blending vessel by spending 80 minutes to carry out the emulsification under the conditions of a temperature of 80 degrees C and a stirring rotational speed of 35 rpm. After that, the solution was cooled to equal to or lower than 30 degrees C with a refrigerant of 5 degrees C to obtain the premixed composition.

In addition to above, the maximum value of the power consumption in the emulsification was directly measured by employing a clamp-type dynamometer (Clamp-on Power High Tester 3169, commercially available from HIOKI E.E. CORPORATION), and the result was 1.78 kW/$m^3$.

(Evaluations of Premixes)

(1) The existence or nonexistence of the vesicle formation in the premix obtained in each of Examples was evaluated through a polarization microscopy.

◎ (double circle): clear Maltese Cross derived from the vesicle structure was observed

○ (circle): Maltese Cross derived from the vesicle structure was observed
× (cross): Maltese Cross of derived from the vesicle structure was not observed.

(2) The mean particle diameter of the vesicle in the premix obtained in each of Examples was determined at 25 degrees C by employing Multisizer™ 4 commercially available from Beckman Coulter, Inc. In addition to above, the mean particle diameter was presented by the volumetric-basis median diameter (D50).

(Preparation of rinse)

Rinses were prepared by employing the premixed compositions obtained in Reference Examples 1 to 20. 301.40 g of an aqueous phase of deionized water and 2.36 g of MUSASHINO LACTIC ACID 90 (lactic acid, degree of purity 90%, molecular weight 90.08: melting point 18 degrees C, commercially available from Musashino Chemical Laboratory Ltd.) were supplied in a 500 mL beaker, and the mixture was heated to 55 degrees C while being stirred with a propeller. Thereafter, an oil phase composed of 9.29 g of FARMIN DM E-80 (N,N-dimethyloctadecyloxy propylamine, degree of purity 90%, molecular weight 355.63, commercially available from Kao Corporation), 21.00 g of KALCOL 8098 (stearyl alcohol, commercially available from Kao Corporation) and 5.95 g of DPG-RF (dipropylene glycol, melting point -40 degrees C, commercially available from ADEKA) was uniformly dissolved at 80 degrees C, and then was added in the aqueous phase, and was stirred at 300 rpm for 10 minutes to carry out emulsification. It was radiationally-cooled to a temperature of equal to or lower than 35 degrees C to prepare a base rinse, and 10.00 g of the aforementioned premixed compositions were added to produce rinses.

(Method for Evaluating Rinses)

20 g of hair strands (about 15.20 cm) provided by Japanese women and treated with cosmetic treatments such as cold-waving, hair-bleaching and the like, were tied together, and was cleaned with the shampoo. This hair was uniformly applied with 2 g of the rinse prepared in each of Reference Examples 1 to 20, and was rinsed off with running water for 30 seconds, and then towel drying was conducted, and further drying was conducted with a hair dryer, and general sensory evaluations of the resultant hair were conducted in relation to: "smoothness and absence of slimy feel in rinsing" and "smoothness and absence of pastiness after drying". The evaluations were made by 5 specialty panelists with 5 scale-evaluations, and were averaged. Products having averaged evaluations of equal to or higher than 3 were classified as acceptable products.

(Criteria for Evaluation)

"the smoothness and the absence of the slimy feel in rinsing"

5: superior in both of the smoothness and the absence of the slimy feel in rinsing
4: better in both of the smoothness and the absence of the slimy feel in rinsing
3: better in either one of the smoothness and the absence of the slimy feel in rinsing
2: inferior in either one of the smoothness and the absence of the slimy feel in rinsing
1: inferior in both of the smoothness and the absence of the slimy feel in rinsing

"the smoothness and the absence of the pastiness after the drying"

5: superior in both of the smoothness and the absence of the pastiness after drying;
4: better in both of the smoothness and the absence of the pastiness after drying;
3: better in either one of the smoothness and the absence of the pastiness after drying;
2: inferior in either one of the smoothness and the absence of the pastiness after drying;
1: inferior in both of the smoothness and the absence of the pastiness after drying.

[Table 6]

TABLE 6

| | | UNIT | REFERENCE EXAMPLE 1 | REFERENCE EXAMPLE 2 | REFERENCE EXAMPLE 3 | REFERENCE EXAMPLE 4 | REFERENCE EXAMPLE 5 |
|---|---|---|---|---|---|---|---|
| FORMULATION OF PREMIX | (A) 18-MEA | [wt%] | 3.50 | - | - | - | - |
| | (A) STEARIC ACID | [wt%] | - | 2.00 | - | - | 1.20 |
| | (A) MYRISTIC ACID | [wt%] | - | - | 2.00 | - | - |
| | (A) BEHENIC ACID | [wt%] | - | - | - | 2.00 | |
| | (A) ISOSTEARIC ACID | [wt%] | - | - | - | - | 0.80 |
| | (B) N,N-DIMETHYLOCTADECYLOXY PROPYLAMINE | [wt%] | 4.74 | 3.47 | 4.32 | 2.90 | 3.44 |
| | (B) N-(3-(DIMETHYLAMINO)PROPYL)DOCOSANAMIDE | [wt%] | - | - | - | - | - |
| | (B) N,N-DIMETHYL-n-OCTADECYLAMINE | [wt%] | - | - | - | - | - |
| | (C) LACTIC ACID | [wt%] | 0.24 | 0.18 | 0.22 | 0.15 | 0.17 |
| | DIPROPYLENE GLYCOL | [wt%] | 10.50 | 6.00 | 6.00 | 6.00 | 6.00 |
| | PURIFIED WATER | [wt%] | 81.02 | 88.35 | 87.46 | 88.95 | 88.39 |
| | PREMIX TOTAL | [wt%] | 100.00 | 100.00 | 100.00 | 100.00 | 100.00 |

(continued)

| | | UNIT | REFERENCE EXAMPLE 1 | REFERENCE EXAMPLE 2 | REFERENCE EXAMPLE 3 | REFERENCE EXAMPLE 4 | REFERENCE EXAMPLE 5 |
|---|---|---|---|---|---|---|---|
| CONDITIONS FOR PREPARING PREMIX | TOTAL LOADING QUANTITY IN BLENDING VESSEL | [kg] | 110 | 110 | 110 | 110 | 110 |
| | EMULSIFICATION METHOD | [-] | PHASE INVERSION | PHASE INVERSION | PHASE INVERSION | PHASE INVERSION | PHASE INVERSION |
| | DEGREE OF NEUTRALIZATION: {(A)+(C)}/(B) | [MOLAR RATIO] | 1.0 | 1.0 | 1.0 | 1.0 | 1.0 |
| | (A) : (C) | [MOLAR RATIO] | 8 : 2 | 8 : 2 | 8 : 2 | 8 : 2 | 8 : 2 |
| | EMULSIFICATION TEMPERATURE | [°C] | 80 | 80 | 80 | 80 | 80 |
| | TYPE OF IMPELLER | [-] | AGI HOMO MIXER | AGI HOMO MIXER | AGI HOMO MIXER | AGI HOMO MIXER | AGI HOMO MIXER |
| | ROTATIONAL SPEED | [rpm] | 35 | 35 | 35 | 35 | 35 |
| | TIME FOR DROPPING AQUEOUS PHASE | [min] | 80 | 80 | 80 | 80 | 80 |
| | POWER CONSUMPSION: PV | [$kW/m^3$] | 2.02 | 1.87 | 1.93 | 1.95 | 2.12 |
| RINSE FORMULATION | DEIONIZED WATER | [g] | 301.40 | 301.40 | 301.40 | 301.40 | 301.40 |
| | MUSASHINO LACTIC ACID 90 | [g] | 2.36 | 2.36 | 2.36 | 2.36 | 2.36 |
| | FARMIN DM E-80 | [g] | 9.29 | 9.29 | 9.29 | 9.29 | 9.29 |
| | KALCOL 8098 | [g] | 21.00 | 21.00 | 21.00 | 21.00 | 21.00 |
| | DPG-RF | [g] | 5.95 | 5.95 | 5.95 | 5.95 | 5.95 |
| | PREMIX | [g] | 10.00 | 10.00 | 10.00 | 10.00 | 10.00 |
| | RINSE TOTAL | [g] | 350.00 | 350.00 | 350.00 | 350.00 | 350.00 |

(continued)

| | | UNIT | REFERENCE EXAMPLE 1 | REFERENCE EXAMPLE 2 | REFERENCE EXAMPLE 3 | REFERENCE EXAMPLE 4 | REFERENCE EXAMPLE 5 |
|---|---|---|---|---|---|---|---|
| EVALUATION RESULTS | EXISTENCE OR NONEXISTENCE OF VESICLE FORMATION (MICROSCOPIC OBSERVATION) | H | ◎ | ◎ | ○ | ○ | ◎ |
| | MEAN PARTICLE SIZE | [$\mu$m] | 6.1 | 7.0 | 7.1 | 7.5 | 6.7 |
| | SMOOTHNESS AND ABSENCE OF SLIMY FEEL IN RINSING (AVERAGE SCORE) | [-] | 5.0 | 4.6 | 4.0 | 4.4 | 4.5 |
| | SMOOTHNESS AND ABSENCE OF PASTINESS AFTER DRYING (AVERAGE SCORE) | [-] | 5.0 | 4.1 | 3.7 | 4.0 | 4.3 |

[Table 7]

TABLE 7

| | | UNIT | REFERENCE EXAMPLE 6 | REFERENCE EXAMPLE 7 | REFERENCE EXAMPLE 8 | REFERENCE EXAMPLE 9 | REFERENCE EXAMPLE 10 |
|---|---|---|---|---|---|---|---|
| FORMULATION OF PREMIX | (A) 18-MEA | [wt%] | - | - | - | - | 3.50 |
| | (A) STEARIC ACID | [wt%] | - | - | 1.60 | 1.80 | - |
| | (A) MYRISTICACID | [wt%] | 1.20 | - | - | - | - |
| | (A)BEHENICACID | [wt%] | - | 1.20 | - | - | - |
| | (A) ISOSTEARIC ACID | [wt%] | 0.80 | 0.80 | 0.40 | 0.20 | - |
| | (B) N,N-DIMETHYLOCTADECYLOXY PROPYLAMINE | [wt%] | 3.95 | 3.10 | 3.45 | 3.46 | - |
| | (B) N-(3-(DIMETHYLAMINO)PROPYL)D OCOSANAMIDE | [wt%] | - | - | - | - | 5.15 |
| | (B) N,N-DIMETHYL-n-OCTADECYLAMINE | [wt%] | - | - | - | - | - |
| | (C) LACTIC ACID | [wt%] | 0.20 | 0.16 | 0.17 | 0.18 | 0.24 |
| | DIPROPYLENE GLYCOL | [wt%] | 6.00 | 6.00 | 6.00 | 6.00 | 10.50 |
| | PURIFIED WATER | [wt%] | 87.85 | 88.74 | 88.38 | 88.36 | 80.61 |
| | PREMIX TOTAL | [wt%] | 100.00 | 100.00 | 100.00 | 100.00 | 100.00 |

(continued)

| | | UNIT | REFERENCE EXAMPLE 6 | REFERENCE EXAMPLE 7 | REFERENCE EXAMPLE 8 | REFERENCE EXAMPLE 9 | REFERENCE EXAMPLE 10 |
|---|---|---|---|---|---|---|---|
| CONDITIONS FOR PREPARING PREMIX | TOTAL LOADING QUANTITY IN BLENDING VESSEL | [kg] | 110 | 110 | 110 | 110 | 110 |
| | EMULSIFICATION METHOD | [-] | PHASE INVERSION | PHASE INVERSION | PHASE INVERSION | PHASE INVERSION | PHASE INVERSION |
| | DEGREE OF NEUTRALIZATION:{(A)+(C)}/(B) | [MOLAR RATIO] | 1.0 | 1.0 | 1.0 | 1.0 | 1.0 |
| | (A) : (C) | [MOLAR RATIO] | 8 : 2 | 8 : 2 | 8 : 2 | 8 : 2 | 8 : 2 |
| | EMULSIFICATION TEMPERATURE | [°C] | 80 | 80 | 80 | 80 | 80 |
| | TYPE OF IMPELLER | [-] | AGI HOMO MIXER | AGI HOMO MIXER | AGI HOMO MIXER | AGI HOMO MIXER | AGI HOMO MIXER |
| | ROTATIONAL SPEED | [rpm] | 35 | 35 | 30 | 30 | 35 |
| | TIME FOR DROPPING AQUEOUS PHASE | [min] | 80 | 75 | 80 | 80 | 75 |
| | POWER CONSUMPSION: PV | [$kW/m^3$] | 2.05 | 2.23 | 1.12 | 1.20 | 1.88 |
| RINSE FORMULATION | DEIONIZED WATER | [g] | 301.40 | 301.40 | 301.40 | 301.40 | 301.40 |
| | MUSASHINO LACTIC ACID 90 | [g] | 2.36 | 2.36 | 2.36 | 2.36 | 2.36 |
| | FARMIN DM E-80 | [g] | 9.29 | 9.29 | 9.29 | 9.29 | 9.29 |
| | KALCOL 8098 | [g] | 21.00 | 21.00 | 21.00 | 21.00 | 21.00 |
| | DPG-RF | [g] | 5.95 | 5.95 | 5.95 | 5.95 | 5.95 |
| | PREMIX | [g] | 10.00 | 10.00 | 10.00 | 10.00 | 10.00 |
| | RINSE TOTAL | [g] | 350.00 | 350.00 | 350.00 | 350.00 | 350.00 |

(continued)

| | | UNIT | REFERENCE EXAMPLE 6 | REFERENCE EXAMPLE 7 | REFERENCE EXAMPLE 8 | REFERENCE EXAMPLE 9 | REFERENCE EXAMPLE 10 |
|---|---|---|---|---|---|---|---|
| EVALUATION RESULTS | EXISTENCE OR NONEXISTENCE OF VESICLE FORMATION (MICROSCOPIC OBSERVATION) | [-] | ○ | ◎ | ○ | ○ | ◎ |
| | MEAN PARTICLE SIZE | [μm] | 6.4 | 7.0 | 7.5 | 5.5 | 8.4 |
| | SMOOTHNESS AND ABSENCE OF SLIMY FEEL IN RINSING (AVERAGE SCORE) | [-] | 4.2 | 4.3 | 3.9 | 3.7 | 4.8 |
| | SMOOTHNESS AND ABSENCE OF PASTINESS AFTER DRYING (AVERAGE SCORE) | [-] | 4.0 | 4.1 | 3.5 | 3.4 | 4.7 |

[Table 8]

TABLE 8

| | | UNIT | REFERENCE EXAMPLE 11 | REFERENCE EXAMPLE 12 | REFERENCE EXAMPLE 13 | REFERENCE EXAMPLE 14 | REFERENCE EXAMPLE 15 |
|---|---|---|---|---|---|---|---|
| FORMULATION OF PREMIX | (A) 18-MEA | [wt%] | 3.50 | 3.50 | 3.50 | 3.50 | 3.50 |
| | (A) STEARIC ACID | [wt%] | - | - | - | - | - |
| | (A) MYRISTIC ACID | [wt%] | - | - | - | - | - |
| | (A) BEHENIC ACID | [wt%] | - | - | - | - | - |
| | (A) ISOSTEARICACID | [wt%] | - | - | - | - | - |
| | (B) N,N-DIMETHYLOCTADECYLOXY PROPYLAMINE | [wt%] | - | 4.74 | 4.74 | 4.74 | 4.74 |
| | (B) N-(3-(DIMETHYLAMINO)PROPYL)DOCOSANAMIDE | [wt%] | - | - | - | - | - |
| | (B) N,N-DIMETHYL-n-OCTADECYLAMINE | [wt%] | 3.64 | - | - | - | - |
| | (C) LACTIC ACID | [wt%] | 0.24 | 0.24 | 0.24 | 0.24 | 0.24 |
| | DIPROPYLENE GLYCOL | [wt%] | 10.50 | 10.50 | 10.50 | 10.50 | 10.50 |
| | PURIFIED WATER | [wt%] | 82.12 | 81.02 | 81.02 | 81.02 | 81.02 |
| | PREMIX TOTAL | [wt%] | 100.00 | 100.00 | 100.00 | 100.00 | 100.00 |

(continued)

| | | UNIT | REFERENCE EXAMPLE 11 | REFERENCE EXAMPLE 12 | REFERENCE EXAMPLE 13 | REFERENCE EXAMPLE 14 | REFERENCE EXAMPLE 15 |
|---|---|---|---|---|---|---|---|
| CONDITIONS FOR PREPARING PREMIX | TOTAL LOADING QUANTITY IN BLENDING VESSEL | [kg] | 110 | 110 | 110 | 110 | 110 |
| | EMULSIFICATION METHOD | [-] | PHASE INVERSION | PHASE INVERSION | PHASE INVERSION | PHASE INVERSION | PHASE INVERSION |
| | DEGREE OF NEUTRALIZATION:{(A)+(C)}/(B) | [MOLAR RATIO] | 1.0 | 1.0 | 1.0 | 1.0 | 1.0 |
| | (A):(C) | [MOLAR RATIO] | 8 : 2 | 8 : 2 | 8 : 2 | 8 : 2 | 8 : 2 |
| | EMULSIFICATION TEMPERATURE | [°C] | 80 | 80 | 80 | 80 | 80 |
| | TYPE OF IMPELLER | [-] | AGI HOMO MIXER | AGI HOMO MIXER | AGI HOMO MIXER | AGI HOMO MIXER | AGI HOMO MIXER |
| | ROTATIONAL SPEED | [rpm] | 35 | 45 | 60 | 25 | 35 |
| | TIME FOR DROPPING AQUEOUS PHASE | [min] | 78 | 80 | 80 | 80 | 45 |
| | POWER CONSUMPSION:PV | [$kW/m^3$] | 1.92 | 3.31 | 4.29 | 0.45 | 1.46 |
| RINSE FORMULATION | DEIONIZED WATER | [g] | 301.40 | 301.40 | 301.40 | 301.40 | 301.40 |
| | MUSASHINO LACTIC ACID 90 | [g] | 2.36 | 2.36 | 2.36 | 2.36 | 2.36 |
| | FARMIN DM E-80 | [g] | 9.29 | 9.29 | 9.29 | 9.29 | 9.29 |
| | KALCOL8098 | [g] | 21.00 | 21.00 | 21.00 | 21.00 | 21.00 |
| | DPG-RF | [g] | 5.95 | 5.95 | 5.95 | 5.95 | 5.95 |
| | PREMIX | [g] | 10.00 | 10.00 | 10.00 | 10.00 | 10.00 |
| | RINSE TOTAL | [g] | 350.00 | 350.00 | 350.00 | 350.00 | 350.00 |

(continued)

| | | UNIT | REFERENCE EXAMPLE 11 | REFERENCE EXAMPLE 12 | REFERENCE EXAMPLE 13 | REFERENCE EXAMPLE 14 | REFERENCE EXAMPLE 15 |
|---|---|---|---|---|---|---|---|
| EVALUATION RESULTS | EXISTENCE OR NONEXISTENCE OF VESICLE FORMATION (MICROSCOPIC OBSERVATION) | [-] | ◎ | ◎ | ◎ | ○ | ◎ |
| | MEAN PARTICLE SIZE | [$\mu$m] | 6.8 | 5.3 | 4.9 | 9.9 | 5.8 |
| | SMOOTHNESS AND ABSENCE OF SLIMY FEEL IN RINSING (AVERAGE SCORE) | [-] | 4.8 | 4.3 | 4.1 | 3.8 | 4.3 |
| | SMOOTHNESS AND ABSENCE OF PASTINESS AFTER DRYING (AVERAGE SCORE) | [-] | 4.8 | 4.1 | 4.0 | 3.6 | 4.3 |

[Table 9]

TABLE 9

| | | UNIT | REFERENCE EXAMPLE 16 | REFERENCE EXAMPLE 17 | REFERENCE EXAMPLE 18 | REFERENCE EXAMPLE 19 | REFERENCE EXAMPLE 20 |
|---|---|---|---|---|---|---|---|
| FORMULATION OF PREMIX | (A) 18-MEA | [wt%] | 3.50 | 3.50 | 3.50 | 3.50 | 3.50 |
| | (A) STE4RIC ACID | [wt%] | - | - | - | - | - |
| | (A) MYRISTIC ACID | [wt%] | - | - | - | - | - |
| | (A) BEHENIC ACID | [wt%] | - | - | - | - | - |
| | (A) ISOSTEARICACID | [wt%] | - | - | - | - | - |
| | (B) N,N-DIMETHYLOCTADECYLOXY PROPYLAMINE | [wt%] | 4.74 | 4.74 | 4.74 | 4.74 | 4.74 |
| | (B) N-(3-(DIMETHYLAMINO)PROPYL)DOCOSANAMIDE | [wt%] | - | - | - | - | - |
| | (B) N,N-DIMETHYL-n-OCTADECYLAMINE | [wt%] | - | - | - | - | - |
| | (C) LACTIC ACID | [wt%] | 0.24 | 0.24 | 0.24 | 0.24 | 0.24 |
| | DIPROPYLENE GLYCOL | [wt%] | 10.50 | 10.50 | 10.50 | 10.50 | 10.50 |
| | PURIFIED WATER | [wt%] | 81.02 | 81.02 | 81.02 | 81.02 | 81.02 |
| | PREMIX TOTAL | [wt%] | 100.00 | 100.00 | 100.00 | 100.00 | 100.00 |

(continued)

| | | UNIT | REFERENCE EXAMPLE 16 | REFERENCE EXAMPLE 17 | REFERENCE EXAMPLE 18 | REFERENCE EXAMPLE 19 | REFERENCE EXAMPLE 20 |
|---|---|---|---|---|---|---|---|
| CONDITIONS FOR PREPARING PREMIX | TOTAL LOADING QUANTITY IN BLENDING VESSEL | [kg] | 700 | 110 | 110 | 110 | 110 |
| | EMULSIFICATION METHOD | [-] | PHASE INVERSION | PHASE INVERSION | PHASE INVERSION | PHASE INVERSION | NORMAL PHASE |
| | DEGREE OF NEUTRALIZATION: {(A)+(C)}/(B) | [MOLAR RATIO] | 1.0 | 1.0 | 1.0 | 1.0 | 1.0 |
| | (A): (C) | [MOLAR RATIO] | 8 : 2 | 8 : 2 | 8 : 2 | 8 : 2 | 8 : 2 |
| | EMULSIFICATION TEMPERATURE | [°C] | 80 | 80 | 80 | 80 | 80 |
| | TYPE OF IMPELLER | [-] | AGI HOMO MIXER | SUPER MIX IMPELLER | AGI HOMO MIXER | AGI HOMO MIXER | AGI HOMO MIXER |
| | ROTATIONAL SPEED | [rpm] | 40 | 90 | 10 | 80 | 35 |
| | TIME FOR DROPPING AQUEOUS PHASE | [min] | 77 | 30 | 71 | 15 | 80 |
| | POWER CONSUMPSION: PV | [kW/m$^3$] | 1.37 | 1.53 | 0.08 | 11.0 | 1.78 |
| RINSE FORMULATION | DEIONIZED WATER | [g] | 301.40 | 301.40 | 301.40 | 301.40 | 301.40 |
| | MUSASHINO LACTIC ACID 90 | [g] | 2.36 | 2.36 | 2.36 | 2.36 | 2.36 |
| | FARMIN DM E-80 | [g] | 9.29 | 9.29 | 9.29 | 9.29 | 9.29 |
| | KALCOL 8098 | [g] | 21.00 | 21.00 | 21.00 | 21.00 | 21.00 |
| | DPG-RF | [g] | 5.95 | 5.95 | 5.95 | 5.95 | 5.95 |
| | PREMIX | [g] | 10.00 | 10.00 | 10.00 | 10.00 | 10.00 |
| | RINSE TOTAL | [g] | 350.00 | 350.00 | 350.00 | 350.00 | 350.00 |

(continued)

| | | UNIT | REFERENCE EXAMPLE 16 | REFERENCE EXAMPLE 17 | REFERENCE EXAMPLE 18 | REFERENCE EXAMPLE 19 | REFERENCE EXAMPLE 20 |
|---|---|---|---|---|---|---|---|
| EVALUATION RESULTS | EXISTENCE OR NONEXISTENCE OF VESICLE FORMATION (MICROSCOPIC OBSERVATION) | [-] | ◎ | ◎ | × | × | × |
| | MEAN PARTICLE SIZE | [μm] | 6.6 | 11.2 | 3.5 | 3.1 | 4.1 |
| | SMOOTHNESS AND ABSENCE OF SLIMY FEEL IN RINSING (AVERAGE SCORE) | [-] | 4.7 | 4.8 | 2.2 | 1.6 | 1.7 |
| | SMOOTHNESS AND ABSENCE OF PASTINESS AFTER DRYING (AVERAGE SCORE) | [-] | 4.7 | 4.7 | 2.0 | 1.3 | 1.5 |

**Claims**

1. A method for producing a vesicle composition having an aqueous phase as a continuous phase, the method comprising:

    an oil phase-preparation step for dissolving the oil phase comprising components (A) to (D) at a temperature of equal to or higher than melting point of the oil phase, said components (A) to (D) being:

        the component (A): a fatty acid of total carbon number of from 12 to 40;
        the component (B): a tertiary amine compound of total carbon number of from 8 to 75;
        the component (C): an organic acid of total carbon number of from 1 to 10; and
        the component (D): the first polyol,

    an emulsification step for adding the aqueous phase to the oil phase obtained in the oil phase-preparation step while being mixed;
    an addition step for adding a component (D'): the second polyol, after a quantity of the aqueous phase added to the oil phase is equal to or larger than 1 time by mass of a quantity of the oil phase; and
    a cooling step for cooling the obtained composition after the emulsification step.

2. The method for producing a vesicle composition according to Claim 1, wherein said mixing is carried out in said emulsification step so that a maximum value of a power consumption Pv is equal to or larger than 0.1 $kW/m^3$ and equal to or smaller than 10 $kW/m^3$.

3. The method for producing a vesicle composition according to Claim 1 or 2, wherein said mixing is carried out in said emulsification step so as to provide total weight of said oil phase and said aqueous phase loaded in a blending vessel as equal to or higher than 10 kg to achieve the phase inversion emulsification.

4. The method for producing a vesicle composition according to any one of Claims 1 to 3, wherein content of said component (D') in said vesicle composition is equal to or higher than 1% by mass and is equal to or lower than 30 % by mass.

5. The method for producing a vesicle composition according to any one of Claims 1 to 4, wherein in said oil phase-preparation step a component (E): a fatty acid ester is further blended in said oil phase.

6. The method for producing a vesicle composition according to any one of Claims 1 to 5, wherein said addition step is carried out after said emulsification step.

7. The method for producing a vesicle composition according to any one of Claims 1 to 6, wherein said component (D') is one compound or two compounds selected from propylene glycol and dipropylene glycol.

8. The method for producing a vesicle composition according to Claim 7, wherein ratio of propylene glycol to dipropylene glycol in said component (D') is from 10:90 to 90:10.

9. The method for producing a vesicle composition according to any one of Claims 1 to 8, wherein a temperature in said mixing in said emulsification step is equal to or higher than a gel transition temperature of an obtainable vesicle composition.

10. The method for producing a vesicle composition according to any one of Claims 1 to 9, wherein in said cooling step the vesicle composition is cooled to a temperature of equal to or lower than a gel transition temperature.

11. The method for producing a vesicle composition according to any one of Claims 1 to 10, wherein total amount of said component (A), said component (B) and said component (C) in said vesicle composition is equal to or higher than 1 % by mass and is equal to or lower than 20 % by mass.

12. The method for producing a vesicle composition according to any one of Claims 1 to 11, wherein said components (A), (B) and (C) satisfies that a ratio of the sum of acid equivalents of said component (A) and said component (C) to a base equivalent of said component (B) is equal to or higher than 0.25 and equal to or lower than 4.

**13.** The method for producing a vesicle composition according to any one of Claims 1 to 12, wherein rotational speed in the emulsification in said emulsification step is equal to or higher than 15 rpm and equal to or lower than 800 rpm.

**14.** The method for producing a vesicle composition according to any one of Claims 1 to 13, wherein a time for dropping the aqueous phase in the emulsification in said emulsification step is equal to or longer than 5 minutes and equal to or shorter than 100 minutes.

**15.** A vesicle composition obtainable by the method for producing a vesicle composition according to any one of Claims 1 to 14.

**16.** A hair cosmetic composition in a form of a treatment, a conditioner or a rinse, obtainable by adding the vesicle composition according to Claim 15 in a hair cosmetic composition base comprising one or more of surfactant(s) and an aliphatic alcohol.

**17.** A method for producing a hair cosmetic composition, comprising:

a hair cosmetic composition base-preparation step for adding an oil phase comprising a surfactant and an aliphatic alcohol in an aqueous phase to carry out an emulsification; and
a vesicle blending step for adding the vesicle composition according to Claim 15 in the composition obtained in the hair cosmetic composition base-preparation step.

**INTERNATIONAL SEARCH REPORT**

International application No.
PCT/JP2013/053397

A. CLASSIFICATION OF SUBJECT MATTER
*A61K8/36*(2006.01)i, *A61K8/14*(2006.01)i, *A61K8/34*(2006.01)i, *A61K8/362*(2006.01)i, *A61K8/365*(2006.01)i, *A61K8/37*(2006.01)i, *A61K8/41*(2006.01)i, *A61K8/44*(2006.01)i, *A61Q5/12*(2006.01)i, *B01J13/04*(2006.01)i
According to International Patent Classification (IPC) or to both national classification and IPC

B. FIELDS SEARCHED
Minimum documentation searched (classification system followed by classification symbols)
A61K8/36, A61K8/14, A61K8/34, A61K8/362, A61K8/365, A61K8/37, A61K8/41, A61K8/44, A61Q5/12, B01J13/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2011/007525 A1 (Kao Corp.), 20 January 2011 (20.01.2011), claims 1 to 10; paragraphs [0050], [0051]; examples 1 to 12 & EP 2455060 A1 & CN 102470084 A & TW 201106981 A | 15-17 |
| Y | WO 2011/122477 A1 (Shiseido Co., Ltd.), 06 October 2011 (06.10.2011), paragraphs [0037], [0038] & JP 2012-92084 A & CN 102811708 A & TW 201201849 A | 15-17 |
| Y | JP 2008-24681 A (Shiseido Co., Ltd.), 07 February 2008 (07.02.2008), paragraphs [0025], [0026] (Family: none) | 15-17 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 March, 2013 (07.03.13) | 19 March, 2013 (19.03.13) |
| Name and mailing address of the ISA/ Japanese Patent Office | Authorized officer |
| Facsimile No. | Telephone No. |

Form PCT/ISA/210 (second sheet) (July 2009)

**INTERNATIONAL SEARCH REPORT**

International application No.
PCT/JP2013/053397

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2007-268526 A (Lion Corp.), 18 October 2007 (18.10.2007), paragraph [0028] (Family: none) | 15-17 |
| A | WO 2004/004676 A1 (Kose Corp.), 15 January 2004 (15.01.2004), claims & JP 4527530 B2 & US 2005/0287095 A1 & CA 2491725 A & HK 1079982 A & AU 2003246269 A & CN 1665478 A | 1-17 |
| A | JP 2007-254405 A (Shiseido Co., Ltd.), 04 October 2007 (04.10.2007), claims; paragraphs [0041], [0042] (Family: none) | 1-17 |
| A | JP 10-180088 A (Lion Corp.), 07 July 1998 (07.07.1998), claims (Family: none) | 1-17 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H04173719 B
- WO 2011007525 A
- JP 2007161683 A


### Non-patent literature cited in the description


- Cosmetics Raw Material Standards. Isostearic acid. YAKUJI NIPPO LIMITED, 1984, 87
- *LIPIDS,* 1988, vol. 23 (9), 878-881